(19) Europäisches Patentamt
European Patent Office
Office européen des brevets

(11) EP 3 626 835 A1

(12) EUROPEAN PATENT APPLICATION

(43) Date of publication:
25.03.2020 Bulletin 2020/13

(21) Application number: 18382671.8

(22) Date of filing: 18.09.2018

(51) Int Cl.:
*C12Q 1/6858* (2018.01) *C12Q 1/6881* (2018.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: Sistemas Genómicos, S.L.
46980 Paterna (Valencia) (ES)

(72) Inventors:
- Valero Hervás, Diana María
46980 Valencia (ES)
- Triviño Pardo, Juan Carlos
46980 Valencia (ES)
- Gómez Ramos, Alberto
46980 Valencia (ES)
- Rosa Ferrer, Ricardo
46980 Valencia (ES)
- Bernad Palomares, Lucía
46980 Valencia (ES)
- Cabo Díez, Miguel
46980 Valencia (ES)
- Collado Micó, Maria Carmen
46980 Valencia (ES)
- Buades Gomis, Celia
46980 Valencia (ES)
- Gorriz Belert, Manuel
46980 Valencia (ES)
- Fernández Pedrosa, María Victoria
46980 Valencia (ES)

(74) Representative: Manuel Illescas y Asociados, S.L.U.
C/ Príncipe de Vergara 197, Oficina 1°A
28002 Madrid (ES)

# (54) METHOD FOR GENOTYPICALLY IDENTIFYING BOTH ALLELES OF AT LEAST ONE LOCUS OF A SUBJECT'S HLA GENE

(57) The method of the invention enables performing a genetic test on patients in order to learn the haplotypes (combination of alleles at the same loci of a chromosome that are transmitted together) of their human leukocyte antigens (HLA) with a precision, accuracy and improved reliability with respect to the methods known in the state of the art. The invention establishes, by bioinformatic analysis, the precise determination of these haplotypes and enables knowing which individuals are compatible for transplants; diagnosis/prognosis of autoimmune diseases; paternity tests; adverse reactions to drugs in personalised medicine; vaccination, etc.

EP 3 626 835 A1

SEQUENCE LISTING

<110> Sistemas Genómicos, S.L.

<120> Method for genotypically identifying both alleles of at least one locus of a subject's HLA gene

<130> EP-07957

<160> 22

<170> BiSSAP 1.3.6

<210> 1
<211> 26
<212> DNA
<213> Artificial Sequence

<220>
<223> HLA-A forward primer

<400> 1
tggactcaca cagaaactca gagcta 26

<210> 2
<211> 28
<212> DNA
<213> Artificial Sequence

<220>
<223> HLA-A reverse primer

<400> 2
atataaccat catcgtgtcc caaggttc 28

<210> 3
<211> 21
<212> DNA
<213> Artificial Sequence

<220>
<223> HLA-B forward primer

<400> 3
tctgatcaca cccccttagaa c 21

<210> 4
<211> 21
<212> DNA
<213> Artificial Sequence

<220>
<223> HLA-B reverse primer

<400> 4
agagggttct ttgcattcgg t 21

<210> 5
<211> 22
<212> DNA
<213> Artificial Sequence

<220>
<223> HLA-C forward primer

<400> 5
attaacatag cctcaggcca ag 22

<210> 6
<211> 22
<212> DNA
<213> Artificial Sequence

<220>
<223> HLA-C reverse primer

<400> 6
agctcactgt ctggcatcaa gt 22

<210> 7
<211> 23
<212> DNA
<213> Artificial Sequence

<220>
<223> HLA-DRB1 forward primer

<400> 7
tcattccaca cattttggtt cta 23

<210> 8
<211> 29
<212> DNA
<213> Artificial Sequence

<220>
<223> HLA-DRB1 reverse primer

<400> 8
gcatccacag aatcacattt tctagtgtt 29

<210> 9
<211> 22
<212> DNA
<213> Artificial Sequence

<220>
<223> HLA-DRB3 forward primer

<400> 9
ggaggccgag ttactgttta ct 22

<210> 10
<211> 23
<212> DNA
<213> Artificial Sequence

<220>
<223> HLA-DRB3 reverse primer

<400> 10
gaataacaaa cctaaagctg ctg 23

<210> 11
<211> 20
<212> DNA
<213> Artificial Sequence

<220>
<223> HLA-DRB4 forward primer

<400> 11
tgcagtacag tccagccttg 20

<210> 12
<211> 22
<212> DNA
<213> Artificial Sequence

<220>
<223> HLA-DRB4 reverse primer

<400> 12
tcatcccaca cattttcgtt ct 22

<210> 13
<211> 26
<212> DNA
<213> Artificial Sequence

<220>
<223> HLA-DRB5 forward primer

<400> 13
gaatcacagc attgtccagt attgaa 26

<210> 14
<211> 24
<212> DNA
<213> Artificial Sequence

<220>

<223> HLA-DRB5 reverse primer

<400> 14
aggaagagtt ctgattgact tgct 24

<210> 15
<211> 24
<212> DNA
<213> Artificial Sequence

<220>
<223> HLA-DPB1 forward primer

<400> 15
cctcctgacc ctgatgacag tcct 24

<210> 16
<211> 26
<212> DNA
<213> Artificial Sequence

<220>
<223> HLA-DPB1 reverse primer

<400> 16
gcacagtagc tttcgggaat tgacca 26

<210> 17
<211> 23
<212> DNA
<213> Artificial Sequence

<220>
<223> HLA-DQB1 forward primer

<400> 17
cacaagaaac aaactgcccc tta 23

<210> 18
<211> 21
<212> DNA
<213> Artificial Sequence

<220>
<223> HLA-DQB1 reverse primer

<400> 18
tgccaggcgt tgttctaatt g 21

<210> 19
<211> 25
<212> DNA
<213> Artificial Sequence

```
<220>
<223> HLA-DPA1 forward primer

<400> 19
agaaactgtt cacagaatgt ccagc                                          25


<210> 20
<211> 20
<212> DNA
<213> Artificial Sequence


<220>
<223> HLA-DPA1 reverse primer

<400> 20
cctgcgatgt gcaggagtta                                                20


<210> 21
<211> 22
<212> DNA
<213> Artificial Sequence


<220>
<223> HLA-DQA1 forward primer

<400> 21
gggacctcaa aaaccctctt ca                                             22


<210> 22
<211> 20
<212> DNA
<213> Artificial Sequence


<220>
<223> HLA-DQA1 reverse primer

<400> 22
gagtctgagt tgggcagcat                                                20
```

## Description

## TECHNICAL FIELD

This invention belongs to the technical field of molecular genotyping. In particular, the technical field relates to genetic identification methods that use sequence amplification by polymerase chain reaction (PCR), Next Generation sequencing and bioinformatic analysis for assigning sequences of the human leukocyte antigens (HLA) to a known HLA family or subfamily or, alternatively, for determining *de novo* sequences.

## BACKGROUND

The human leukocyte antigen (HLA) system is controlled by genes located on the short arm of chromosome 6 and is one of the most polymorphic genetic systems in humans. The specific set of HLA molecules expressed by an individual determines the antigenic repertoire against which the cells of the immune system of each individual can respond, including the recognition of tissues or molecules as their own or as foreign, making it the system responsible for compatibility in organ transplants or the development and control of pathologies of infectious and autoimmune origin. There are 3 types of HLA molecules.

HLA class I molecules are transmembrane glycoproteins present in all the nucleated cells of the organism. These glycoproteins consist of an alpha chain encoded by each of the HLA class I genes linked non-covalently to a molecule of microglobulin 2. Their function is to present protein antigens of proteins produced inside the cells to the cytotoxic T lymphocytes or CD8 (hereafter this will be referred to as the cytosolic or endogenous pathway). The cells that present fragments of foreign proteins will be attacked by the immune system.

The class II molecules are present in professional antigen-presenting cells such as dendritic cells, B lymphocytes and mononuclear phagocytes. At the molecular level, these proteins consist of 2 polypeptide chains encoded by genes located on the HLA-DP, -DQ or -DR regions of chromosome 6. The T helper or CD4 lymphocytes recognise the molecules of type II on the surface of antigen presenting cells, thus becoming activated. The HLA type II antigens presented by the cells come from extracellular proteins (hence this pathway is called exogenous or endocytic).

The HLA class II molecules encode, apart from other products, several secreted proteins that perform immune functions, including components of the complement system and molecules related to inflammation.

The HLA genes are closely linked in such a way that they are inherited in a block, as haplotypes of each parent in a Mendelian way. The random combinations of different HLA loci in a haplotype are very high; however, certain haplotypes are much more frequently represented in the population than others, in a higher proportion than would be expected by chance (linkage disequilibrium). The molecules of HLA class I and II are the most immunogenic antigens that are recognized during rejection of an allogenic transplant. The strongest determinant is HLA-DR, followed by HLA-B and -A. Currently, official reference guides and consortia for the establishment of the criteria required to proceed to HLA typing, establish the determination of the HLA-A, HLA-B and HLA-DRB1 as a minimum requirement for the Solid Organ Transplantation (SOT) and HLA-A, HLA-B, HLA-C, HLA-DRB1, HLA-DQB1, HLA-DPB1 alleles at the high resolution level for Hematopoietic Stem Cell Transplantation (HSCT).

The precise determination of both class I and class II HLA haplotypes in an individual is, therefore, a key fact for the determination of donor-recipient compatibility in the case of organ or tissue transplants.

Methods for genotyping HLA alleles have been described in the prior art. The most representative documents of the state of the art are listed and their technical content is discussed below.

Document EP3006571A1 refers to a method for determining the sequence of HLA molecules by long-range PCR using primers designed to hybridize with upstream and downstream regions of at least two genes selected from HLA-A, HLA-B, HLA-C, HLA-DQA1, HLA-DQB1, HLA-DPA1, HLA-DPB1, HLA-DRB1, HLA-DRB3, HLA-DRB4 and HLA-DRB5, amplify at least two genes simultaneously, sequence and search for homologies comparing with the IMGT/HLA database. This database is part of the international ImMunoGeneTics project (IMGT) and specializes in the human major histocompatibility complex (MHC) sequences and includes the HLA system reference sequences named by the World Health Organization (WHO) for HLA system factors. Document EP2735617A1 describes a method similar to the above for determining the HLA sequence by means of long-range PCR in which some alternative primers to those described in EP3006571A1 are used.

Document WO2015/200701A2 refers to a method for determining the sequence of HLA genes by means of long-range PCR using primers that allow the amplification of the complete gene selected outside the regions of high variability, using a mixture of natural nucleotides and nucleotide analogues and performing a deconvolution analysis to determine the genotype of each allele.

WO2014/116729A2 describes a method for genotyping HLA alleles comprising the amplification stages of exons and introns of genes in a long-range PCR reaction, deep sequencing of the amplified gene and performing a deconvolution analysis to determine the genotype of each allele.

Document WO2014/065410A1 refers to a method comprising PCR amplification using primers that specifically hybridize to the upstream and downstream region of the HLA-A, HLA-B, HLA-C, HLA-DRB1, HLA-DPB1 and HLA-DQB1 genes and performing a homology search with respect to the IMGT/HLA database.

The document by Hosomichi, K. et al. (2013) discloses the application of long-range PCR to amplify six complete HLA genes (HLA-A/B/C, DRB1, DQB1 and DPB1) using primers that bind to conserved areas of the genome, followed by the construction of libraries and multiplex sequencing of said libraries.

Document EP2599877A1 describes a method in which HLA gene amplification is performed with specific primers, followed by fragmentation of the obtained amplicons, obtaining of libraries and alignment of the sequences obtained after sequencing with respect to the IMGT/HLA database.

Document WO2016/054135A1 describes a method for genotyping both alleles of HLA loci in which amplicons are formed, fragmented, sequenced by sequencing-by-synthesis, the partially overlapping nucleotide sequences are aligned to determine an adjoining composite sequence coding for antigen recognition domains (ARD), nucleotide sequences that partially overlap with a reference library of genomic sequences encoding ARD of HLA loci are compared and each sequence is identified as a sequence encoding a known ARD of an HLA locus or a sequence encoding a new ARD from an HLA locus. The method is aimed at genotyping the HLA-A/B/C genes.

Document WO2014/150924A2 describes a computerised method for genotyping HLA genes. In the method, relevant alleles are identified by the decomposition of the alleles in exons, the Hamming distances (HD) between the exons and the reads that best fit these exons are calculated.

The document by Shiina, T. et al. (2012) describes a method of molecular genotyping based on Next Generation sequencing in which alleles are determined for some HLA genes. In this method, primers were designed to amplify the complete genes, including the promoter regions and the UTR regions, with the aim of reducing the ambiguities in the determination of HLA alleles.

Document WO2014/066217A1 describes a method comprising amplifying DNA sequences, sequencing them by Next Generation sequencing and determining the identity of said amplified sequences. The determination of said identity is performed by means of a computerised method comprising the following steps: dividing each reading of sequences into one or two haplotypes, based on the application of a k-means algorithm or an expectation-maximization grouping algorithm (expectation-maximization clustering).

Document EP3075863A1 describes a method in which a region from exon 2 to a portion of exon 4 of HLA class II genes is amplified and subjected to DNA genotyping using Next Generation sequencing. In the method, primers were designed to specifically amplify the HLA-DRB1/3/4/5, HLA-DQB1 and HLA-DPB1 genes.

## SUMMARY OF THE INVENTION

The problem of the state of the art is to improve the precision, accuracy and reliability of the methods of determining HLA haplotypes that use sequence amplification by PCR, Next Generation sequencing and bioinformatic analysis of the results obtained in the sequencing.

The solution to this objective technical problem, according to the present invention, is a method to determine HLA haplotypes that combines the following technical characteristics for the determination of the haplotypes of the 11 HLA genes: use of specific combinations of primers, hybridization of these specific combinations of primers in specific upstream and downstream regions of those genes and amplification in a single amplicon of complete genes, complete UTR regions and upstream and downstream regions of the genes and specific steps of bioinformatic analysis comprising variant calling, local alignment of the sequence of the haplotype blocks, pre-calculation of distances, selection of candidate alleles, phylogenetic study, generation of the phylogenetic tree using a neighbour-joining strategy, test of contrast and assignment of levels and families of the target haplotype or, alternatively, determination of HLA haplotypes *de novo.* The invention is based on this new and inventive combination of characteristics.

The advantages provided by the new and inventive combination of these characteristics consist of an improvement in the precision, accuracy and reliability in the determination of HLA haplotypes. Thus, in the examples of the invention (see Example 10) a target sequence that resembles a known HLA family is described, so that known methods of the state of the art would erroneously assign this known HLA family to the target sequence. However, the method of the invention enables determining that this target sequence does not belong to said known family, but is a *de novo* sequence.

Hybridization of specific primer combinations in specific upstream/downstream regions of the genes and amplification in a single amplicon of complete genes, complete UTR regions and upstream/downstream regions of the genes performs more accurate allele typing as it provides more information relative to the genetic profile contained in each of the candidate alleles, allowing a more accurate high resolution assignment. It also allows the identification and description of new previously undescribed alleles by considering a greater content of genetic information, including said upstream and downstream regions of the genes in the amplification step, together with the complete genes, also including the UTR regions. The fact that more information about the sequence is available avoids erroneous assignments since the necessary information to distinguish between two alleles with high homology in the coding sequence but different in the remaining regions, which can affect the resulting protein and therefore the genetic allele to the protein and the function of the HLA molecule in the sequence, can be found in the intronic regions, UTRs or upstream/downstream regions.

These upstream/downstream regions have a lower content in polymorphic variants, thus allowing more specific oligonucleotide designs for the target gene to be performed, avoiding non-specific amplifications, amplifications of pseudogenic regions and possible allele dropout phenomena, which could imply erroneous allele assignments. Allelic loss phenomena occur when an allele is lost during DNA amplification by PCR. For the purposes herein, the terms primer/s and oligonucleotide/s are used interchangeably as synonyms.

The method of the invention enables performing a genetic test on patients in order to learn the haplotypes (combination of alleles at the same loci of a chromosome that are transmitted together) of their human leukocyte antigens (HLA) with a precision, accuracy and improved reliability with respect to the methods known in the state of the art. The invention establishes, by bioinformatic analysis, the precise determination of these haplotypes and enables knowing which individuals are compatible for transplants; diagnosis/prognosis of autoimmune diseases; paternity tests; adverse reactions to drugs in personalised medicine; vaccination, etc.

**DEFINITIONS**

For the purposes herein, the following technical terms are defined as follows:

*Neighbour-joining:* Method described in Felsenstein, J. (1981), Saitou, N. et al. (1987) and Studier, J. A. et al. (1988). Phylogenetic reconstruction method that groups the sequences based on genetic distances according to the balanced minimum evolution criterion (BME), in which the best phylogenetic tree is one that minimises the length of the internal branches. With this method, a new node is created in each step, and finally, a unique phylogenetic tree is obtained, which is considered to be the one that best fits the data. From a star phylogenetic tree, the pair of nearest sequences is determined and joined by an internal node. This process is repeated with the other sequences until they are all joined by internal nodes that minimise the length of each of the internal branches.

Variant calling: Variant calling from Next Generation sequencing data refers to a group of computational techniques for identifying the existence of single nucleotide variants (SNV) from the results of Next Generation sequencing experiments. These computational techniques are alternatives to experimental methods based on single nucleotide polymorphisms (SNPs), known throughout the population. Variant calling is used in the genotypic identification of SNP, with a wide variety of algorithms designed for specific experimental designs and applications. These techniques have been successfully applied in the identification of rare SNPs within a population and to detect somatic SNVs within a subject using multiple tissue samples.

Silhouette: Silhouette refers to a method of interpretation and validation of consistency within groups of data. The technique provides a graphic representation of the situation of an object within its group. The silhouette value is a measure of the similarity of an object to its own group (cohesion) compared to other groups (separation). The silhouette value varies from -1 to +1, wherein a high value indicates that the object is well-matched in its own group and is little related to neighbouring groups. If most objects have a high value, then the grouping configuration is appropriate. If many points have a low or negative value, then the grouping configuration can have too many or too few groups. The silhouette can be calculated with any distance measure such as the Euclidean distance or Manhattan distance.

Wilcoxon contrast: The Wilcoxon signed-rank test is a non-parametric test to compare the average range of two related samples and determine if there are differences between them. It is used as an alternative to the Student t-test when the normality of these samples cannot be assumed. It owes its name to Frank Wilcoxon, who published it in 1945. It is a non-parametric comparison test of two related samples and therefore does not need a specific distribution. It uses the ordinal level of the dependent variable instead. It is used to compare two related measurements and determine if the difference between them is due to chance or not (in the latter case, if the difference is statistically significant). It is used when the underlying variable is continuous but no particular type of distribution is assumed.

Long-range PCR refers to the amplification of DNA lengths that normally cannot be amplified using routine PCR methods or reagents. For simple DNA templates, polymerases optimized for long-range PCR can amplify up to 30 kb and more. For complex genomes, a typical objective is 20 kb.

Contrast test: Test to compare the average range of two related samples and determine if there are differences between them.

Phred scale: A Phred value is a measure of the quality of nucleotide identification generated by automatic DNA sequencing. It was originally developed to assist in the automation of DNA sequencing in the Human Genome Project. Phred quality values are assigned to each call to a nucleotide base in sequences obtained in an automated way. Phred quality values are widely accepted to characterize the quality of DNA sequences and can be used to compare the effectiveness of different sequencing methods. Perhaps the most important use of Phred quality values is automatic determination based on the quality of consensus sequences.

BWA algorithm: *Burrows-Wheeler Aligner,* a bioinformatic tool (algorithm) for mapping short sequences of nucleotides to a reference genome.

Mem algorithm: Algorithm for string search matching. Given two strings, mem are common substrings that cannot extend to the left or to the right without causing a mismatch.

Binary Alignment Map (BAM) is the comprehensive raw data of genome sequencing; it consists of the lossless, compressed binary representation of the Sequence Alignment Map.

BAM file: The Binary Alignment Map (BAM) is the complete unprocessed information of genome sequencing; it consists of the lossless and compressed binary representation of the Sequence Alignment Map (SAM). BAM is the compressed binary representation of SAM.

WhatsHap Software: WhatsHap is a computer program to determine the phase of genomic variants using DNA sequencing reads, a process called haplotype assembly. It is especially suitable for long reads, but also works well with short reads.

SAMtools: Set of tools for the selection of specific regions within a genome and for the indexing of the .bam files. Version 1.2 of SAMtools is used herein. The SAMtools toolkit interacts with the subsequent processing of short alignments of DNA sequence reads in the SAM (sequence alignment map), BAM (binary alignment map) and CRAM formats. These files are generated as output by short reading alignment tools, such as BWA. The set contains tools for variant calling, alignment visualization, classification, indexing, data extraction and format conversion. SAM files can be very large, so compression is used to save space. CRAM files have a restructured binary container format oriented to columns. SAMtools makes it possible to work directly with a compressed BAM file, without having to decompress the entire file.

Bcftools software: The SAMtools mpileup command produces a pileup format file (or BCF) that provides, for each genomic coordinate, the overlapping and indels reading bases in that position in the input BAM files. This can be used for calls of single nucleotide polymorphisms (SNP), for example.

Electropherogram: An electropherogram is a graph made with the results of an electrophoresis analysis. Electropherograms can be produced with results derived from: DNA genealogical tests, paternity tests, DNA sequencing, genetic fingerprinting. The electropherogram shows the sequence of data produced by an automatic DNA sequencing machine.

PCR Master Mix (PCRMM): PCRMM is a premixed concentrated solution that has all the components for a real-time PCR reaction that are not sample specific. The master PCRMM mix generally contains a thermostable DNA polymerase, dNTPs, $MgCl_2$ and additives in a buffer optimized for PCR. It is only necessary to add the DNA template, the primers, the probes (if used) and water to make up the desired reaction volume. One or more probes for fluorescent detection of product formation can be included in real-time quantitative PCR (qPCR).

Massive sequencing: For the purposes of this patent specification, this technical term is synonymous and is used interchangeably with the term "Next Generation Sequencing (NGS)".

Tagmentation. Process in which transposase enzymes randomly cut DNA into short fragments (labels, or tags). Simultaneously, the enzyme adds specific sequences at the ends of the fragmented DNA that will allow the incorporation of the adapters in the following PCR amplification process.

Allele: Each one of the alternative forms of the same gene that are different in their sequence and that can be manifested in specific modifications of the function of that gene (produce variations in inherited characteristics such as, for example, eye colour or group blood). Since most mammals are diploid, they have two sets of chromosomes, one from the father and the other from the mother. Each pair of alleles is located in the same locus or place of the chromosome.

Locus: A fixed position on a chromosome, which determines the position of a gene or a marker (genetic marker). In biology and, by extension, in evolutionary computation, it is used to identify positions of interest on certain sequences. A variant of the DNA sequence located at a given locus is called an allele. The ordered list of known loci for a particular genome is called the genetic map, while the process of determining the locus of a given biological character is called genetic mapping. Diploid and polyploid cells, whose chromosomes have the same allele at some locus, are called homozygotes, while those with different alleles at a locus are heterozygotes.

Haplotype: A combination of alleles from different loci of a chromosome that are transmitted together. A haplotype can be a locus, several loci, or an entire chromosome depending on the number of recombination events that have occurred between a given set of loci. A second meaning of the term, haplotype is a set of single-nucleotide polymorphisms (SNPs) on a particular chromosome that are statistically associated. For the purposes of this patent specification the terms "haplotype" and "haplotype block" are considered synonymous and are used interchangeably.

*de novo* HLA haplotype: An HLA haplotype that belongs to a family that is not contained in the version of the reference database that contains genomic sequences of the HLA alleles that have been used (for example, the reference database IPD-IMGT/HLA (https://www.ebi.ac.uk/ipd/imgt/hla/)).

P7 and P5 indexes: Nucleotide sequence included in the amplification oligonucleotides that allow the unambiguous identification of a sample when several samples are sequenced at the same time.

## DETAILED DESCRIPTION OF THE INVENTION

This invention provides a method for genotypically identifying both alleles of at least one locus of a subject's HLA gene, that comprises:

(a) amplifying genomic DNA samples of said subject by long-range PCR using at least one pair of sense and antisense primers for each sample wherein said pair of sense and antisense primers is identified by the sequences selected from the group that consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, wherein said pair of primers amplifies in a single amplicon at least one complete HLA gene together with 5'-UTR and 3'-UTR regions of said HLA gene, an upstream region and a downstream region of said HLA gene, of both alleles, of at least one locus of an HLA gene, thus forming amplicons, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1,

(b) combining the amplicons from each of the samples,

(c) fragmenting the amplicons, thus obtaining fragments and labelling said fragments, thus forming libraries of labelled fragments,

(d) combining said libraries of labelled fragment,

(e) sequencing said libraries of labelled fragments by Next Generation sequencing, thereby generating reads of partially overlapping sequences,

(f) aligning said sequence reads against a reference genome,

(g) identifying variants based on variant calling,

(h) determining haplotype blocks based on the alignment of the sequences reads against a reference genome and adding flanking regions of said haplotype blocks to said haplotype blocks,

(i) locally aligning the sequence reads of the haplotype blocks obtained in the previous step against all the alleles of a reference database of genomic sequences encoding HLA genes for each HLA gene, thus obtaining first candidate alleles,

(j) pre-calculating the genetic distances and determining the phylogenetic grouping of the HLA families at different levels in the reference database of genomic sequences that encode HLA genes, thus obtaining a distance matrix containing the distance values of each allele with itself and with the others,

(k) carrying out a primary selection of said first candidate alleles based on the overall homology contrast result of the sequence reads to the haplotypes, thus obtaining second candidate alleles,

(l) carrying out a study of evolutionary phylogeny of said second candidate alleles, thus obtaining a phylogenetic tree, accessing the distance matrix obtained in step (j) and selecting HLA alleles from the reference database of genomic sequences encoding the phylogenetically closest HLA genes, thus obtaining contrasted HLA alleles and

(m) carrying out a contrast test with contrasted HLA alleles, calculating the distances between the sequence reads and the HLA alleles contrasted in each level and assigning each reading of sequences to a known HLA family at different levels, wherein a sequence reading is assigned to a known HLA family at different levels when the statistical significance obtained in said contrast test is above a defined cut-off value and wherein a sequence reading is assigned as a *de novo* sequence that codes for a new locus of the HLA gene when the statistical significance obtained in said contrast test is below a defined cut-off value.

Table 1 lists the HLA genes that amplify the pairs of sense and antisense primers identified by SEQ ID NO: 1-22 and information on said primers is provided.

Table 1. Primers, identified by SEQ ID NO: 1-22, that amplify HLA genes

| | | SEQ ID NO | Length | % guanine-cytosine (GC) | UTR | Upstream (bp) | Downstream (bp) |
|---|---|---|---|---|---|---|---|
| HLA-A | Sense | 1 | 26 | 46 | Complete | 774 | |
| | Antisense | 2 | 28 | 43 | Complete | | 1291 |
| HLA-B | Sense | 3 | 21 | 47.62 | Complete | | 1827 |
| | Antisense | 4 | 21 | 47.62 | Complete | 1027 | |
| HLA-C | Sense | 5 | 22 | 45.5 | Complete | | 638 |
| | Antisense | 6 | 22 | 50 | Complete | 510 | |
| HLA-DRB1 | Sense | 7 | 23 | 35 | Complete | | 868 |
| | Antisense | 8 | 29 | 38 | Complete | 505 | |
| HLA-DRB3 | Sense | 9 | 22 | 50 | Complete | 1982 | |
| | Antisense | 10 | 23 | 39 | Complete | | 1122 |
| HLA-DRB4 | Sense | 11 | 20 | 55 | Complete | 1320 | |
| | Antisense | 12 | 22 | 41 | Complete | | 872 |
| HLA-DRB5 | Sense | 13 | 26 | 38 | Complete | 591 | |
| | Antisense | 14 | 24 | 42 | Complete | | 297 |
| HLA-DPB1 | Sense | 15 | 24 | 58 | Complete | | 535 |
| | Antisense | 16 | 26 | 50 | Complete | 1434 | |
| HLA-DQB1 | Sense | 17 | 23 | 43 | Complete | | 699 |
| | Antisense | 18 | 21 | 48 | Complete | 1204 | |
| HLA-DPA1 | Sense | 19 | 25 | 44 | Complete | | 1350 |
| | Antisense | 20 | 20 | 55 | Complete | 1779 | |
| HLA-DQA1 | Sense | 21 | 22 | 50 | Complete | | 2811 |
| | Antisense | 22 | 20 | 55 | Complete | 3188 | |

In one embodiment of the method, after sequencing said libraries of labelled fragments by Next Generation sequencing in step (e), the quality values of the raw sequencing data are determined, wherein said determination is a determination of the quality of the raw data sequences. The quality values are the values of probability of error per base. These quality values can be presented under a Phred scale where a value of 20 means that there is a probability of 1/100 that the base was the product of error.

In one embodiment of the method, after aligning said sequence reads against a reference genome in step (f), filtering is carried out, in which sequences that introduce biases and noise are eliminated.

In one embodiment of the method, after aligning said sequence reads against a reference genome in step (f), low quality sequences and sequences corresponding to PCR duplicates are eliminated.

In one embodiment of the method, the identification of variants based on variant calling of step (g) is carried out based on the analysis of the results of the alignments of the sequence reads. In said identification of variants, the mismatches identified between the sequence reads and the reference genome are analysed to identify real variants of the sample. The identification of variants can be performed by using the GATK algorithm (Genome Analysis Tool Kit) of the Broad Institute (https://software.broadinstitute.org/gatk/) (McKenna, A. et al. (2010)).

In one embodiment of the method, in the determination of haplotype blocks based on the alignment of the sequence reads against a reference genome of step (h), the fragments of the sequences of the alleles to be determined that have specific variants are determined, discriminating between the different sequences of each of the alleles in case of being heterozygous or, in the case of homozygosis, a single sequence with its variants with respect to the reference. The determination of haplotype blocks is based on the alignment of the reads against the reference genes and the identification of previously determined variants on this sequence.

In one embodiment of the method, in the addition of flanking regions of said haplotype blocks to said haplotype blocks of step (h), said flanking regions are homozygous. The addition of homozygous flanking regions facilitates the identification process of the allele. Increasing the size of the sequence reduces ambiguity.

In one embodiment of the method, the pre-calculation of the genetic distances and determination of the phylogenetic grouping of the HLA families at different levels in the reference database of genomic sequences encoding the HLA genes of step (j) is carried out by calculating genetic distances and their evolutionary relationship using the neighbour-joining method (Saitou, N. et al. (1987)). The final result of this stage is a distance matrix of size NxN, wherein the phylogenetic distances of all the sequences from all the others are established and where N is the number of alleles contained in the HLA allele reference database (e.g. the IPD-IMGT/HLA database (https://www.ebi.ac.uk/ipd/imgt/hla/)). The distance matrix is a matrix that contains the distance values of each allele with itself and with the others.

In one embodiment of the method, after step (j) of pre-calculating the genetic distances and determining the phylogenetic grouping of the HLA families at different levels in the reference database of genomic sequences encoding HLA genes, the quality of the families is evaluated from the phylogenetic point of view using the silhouette method (Rousseeuw, PJ (1987)). The silhouette method can evaluate if the distances or phylogenetic relationships between members of the same family are statistically narrower compared to the other of the families in the database. The silhouette value varies between -1 and 1; the closer it is to 1, the more defined is the group. Normally, values above 0.5 are considered acceptable groups.

In one embodiment of the method, in step (k) a primary selection of said first candidate alleles is carried out based on the overall homology contrast result of the sequence reads to the haplotypes, thus obtaining second candidate alleles and the fasta36 algorithm (Pearson, W. R. (2016)) can be used in this global homology contrast. The global contrast enables evaluating the proximity of the identified haplotype(s) using the whole length as a unit, avoiding similarity by minor domains or profiles. The selection of a small group of possible HLAs, the second candidate alleles, is based on a combination of parameters, such as those that define the homology, the percentage of identity and the length of the alignment.

In one embodiment of the method, in step (l) a study of evolutionary phylogeny of said second candidate alleles is carried out, thus obtaining a phylogenetic tree, accessing the distance matrix obtained in step (j) and selecting HLA alleles from the reference database of genomic sequences encoding the phylogenetically closest HLA genes, thus obtaining contrasted HLA alleles. In said stage (l), in the study of evolutionary phylogeny of said second candidate alleles, the R platform and the phangorn library can be used (Schliep K. P. (2011); Schliep, K. P. et al. (2017)). In this stage (l), starting from the second candidate alleles and the haplotype or haplotypes associated with the target samples, a genetic distance calculation is carried out in an "all against all" context, using the F81 strategy (Li, H. et al. (2009)) as a model for describing the genetic distance, a phylogenetic tree is generated using the neighbour-joining strategy (Felsenstein, J. (1981); Saitou, N. et al. (1987); Studier, J. A. et al. (1988)) and the distance matrix is accessed using the cophenetic method (Gascuel, O. (1997)).

In one embodiment of the method, in step (m) a contrast test is carried out with contrasted HLA alleles, the distances between the sequence reads and the contrasted HLA alleles in each level are calculated and each sequence reading is assigned to a known HLA family at different levels, wherein a sequence reading is assigned to a known HLA family at different levels when the statistical significance obtained in said contrast test is above a defined cut-off value and wherein a sequence reading is assigned as a *de novo* sequence that codes for a new locus of the HLA gene when the statistical significance obtained in said contrast test is below a defined cut-off value. This contrast test begins with the contrasted HLA allele, which is the phylogenetically closest HLA allele obtained from the IPD-IMGT/HLA database (https://www.ebi.ac.uk/ipd/imgt/hla/) and that is obtained from the study of evolutionary phylogeny and the target haplotype. Subsequently, the allocation of the family and the different levels of the target haplotype are equalled to the contrasted HLA. Subsequently, one moves from the upper level to the lower level of the contrasted HLA sequence. At each level, the average and median distances and confidence intervals of each distance distribution of the members of the family with identical level are evaluated. If the distance between the target haplotype and the closest contrasted HLA is within the theoretical distribution of the level studied with a significance of 95%, it is considered to belong to that level in a statistically significant way (Sneath, P. H. A. et al. (1973); Bauer, D. F. (1972)). The test ends with a statistical identification of the target haplotype with the different levels. It is considered that the target haplotype was the contrasted allele if the 3 upper levels are statistically significant. If that premise is not fulfilled, the process is repeated with the second HLA of higher homology, and thus recurrently until completing the process with all the HLA alleles obtained in stage (i).

In one embodiment of the method, said statistical significance is calculated based on the statistical value p.

In one embodiment of the method, said defined cut-off value is calculated based on the statistical value p and has a value of $1^{-6}$.

In one embodiment of the method, the length of the fragments generated in step (c) is between 1200 and 1500 bp.

In this patent specification, the term "bp" refers to "base pairs" and both terms are interchangeable.

In one embodiment of the method, the libraries are sequenced in step (e) by Next Generation sequencing with a reading length between 200 and 300 bp.

The invention also provides a pair of primers identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, for use in the genotypic identification of both alleles of at least one locus of a subject's HLA gene, wherein said pair of primers amplifies in a single amplicon a complete HLA gene together with 5'-UTR and 3'-UTR regions of said HLA gene, an upstream region and a downstream region of said HLA gene, of both alleles of at least one locus of an HLA gene, wherein said HLA gene is selected, respectively, from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1.

The invention also provides a kit for genotypically identifying both alleles of at least one locus of a subject's HLA gene, comprising:

(a) at least one pair of sense and antisense primers, identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, wherein said pair of primers amplifies in a single amplicon a complete HLA gene together with 5'-UTR and 3'-UTR regions of said HLA gene, an upstream region and a downstream region of said HLA gene, of both alleles of at least one locus of the HLA gene, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1,

(b) a DNA polymerase and

(c) instructions for use.

In one embodiment, the kit additionally comprises reagents for amplification and sequencing.

In one embodiment, the kit is for use in the genotypic identification of both alleles of at least one locus of an HLA gene, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1.

The invention also provides a computerised product comprising a computer readable medium, wherein there are coded instructions for controlling a computerised system comprising the following means for carrying out a genotypic identification of both alleles of at least one locus of a subject's HLA gene, wherein said genotypic identification comprises amplifying genomic DNA samples of said subject by long-range PCR using at least one pair of sense and antisense primers, for each sample, wherein said pair of sense and antisense primers is identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, wherein said pair of primers amplifies in a single amplicon at least one complete HLA gene together with 5'-UTR and 3'-UTR regions of said HLA gene, an upstream region and a downstream region of said HLA gene, of both alleles, of at least one locus of an HLA gene, thus forming amplicons, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1, combining the amplicons for each of the samples, fragmenting the amplicons, thus obtaining fragments and labelling said fragments, thus forming libraries of labelled fragments, combining said libraries of labelled fragments, sequencing said libraries of labelled fragments by Next Generation sequencing, thus generating sequence reads that partially overlap and where such a system comprises the following means:

(a) means for carrying out an alignment of said sequence reads against a reference genome,

(c) means for identifying variants based on a variant calling,

(d) means for determining haplotype blocks based on the alignment of sequence reads against a reference genome and means for adding flanking regions of said haplotype blocks to said haplotype blocks,

(e) means for locally aligning the sequence reads of the haplotype blocks obtained in the previous step against all the alleles of a reference database of genomic sequences encoding HLA genes for each HLA gene, thus obtaining first candidate alleles,

(f) means for pre-calculating the genetic distances and determining the phylogenetic grouping of the HLA families at different levels in the reference database of genomic sequences that encode HLA genes, thus obtaining a distance matrix containing the distance values of each allele with itself and with the others,

(g) means for carrying out a primary selection of said first candidate alleles based on the overall homology contrast result of the sequence reads to the haplotypes, thus obtaining second candidate alleles,

(h) means for carrying out an evolutionary phylogeny study of said second candidate alleles, thus obtaining a phylogenetic tree, means for accessing the distance matrix obtained in step (f) and means for selecting HLA alleles from the reference database of genomic sequences that encode phylogenetically closest HLA genes, thereby obtaining contrasted HLA alleles,

(i) means for carrying out a contrast test with contrasted HLA alleles and

(j) means for calculating the distances between the sequence reads and the contrasted HLA alleles at each level and means for assigning each sequence reading to a known HLA family at different levels, wherein a sequence reading is assigned to a known HLA family at different levels when the statistical significance obtained in said contrast test is above a defined cut-off value and wherein a sequence reading is assigned as a *de novo* sequence that codes for a new locus of the HLA gene when the statistical significance obtained in said contrast test is below a defined cut-off value.

In one embodiment of the computer product, said statistical significance is calculated based on the statistical value p.

In one embodiment of the computer product, said statistical significance is calculated based on the statistical value p and has a value of $1^{-6}$.

**Next Generation sequencing**

It is possible to use any suitable sequencing method to carry out the methods described in this document. In some embodiments, a high throughput sequencing method is used. In high-throughput sequencing methods, the DNA sequences are generally sequenced massively and in parallel within a flow cell. The lane is the basic physical unit in a Next Generation sequencing platform and refers to the basic independent execution in said Next Generation sequencing platform. High-throughput sequencing technologies include sequencing-by-synthesis.

The systems used for high-throughput sequencing methods are commercially available and include, for example, the MiniSeq®, MiSeq®, NextSeq® 550, HiSeq® 2000, HiSeq® 2500, HiSeq® 3000, HiSeq® 4000 and NovaSeq® platforms from Illumina, Inc. and the ION Proton and ION PGM sequencing platforms from ThermoFisher.

In sequencing-by-synthesis, millions of nucleic acid fragments (e.g. DNA) can be sequenced in parallel. In one example of this type of sequencing technology, a flow cell containing an optically transparent slide with 2 individual lanes is used on which oligonucleotides (e.g. adapter oligonucleotides) are attached to the surface. One flow cell is often a solid support that can be configured to retain and/or allow the orderly passage of the reagent solutions onto the bound analytes. Flow cells often have a flat, optically transparent shape, usually in the millimetre or sub-millimetre scale, and often have channels or lanes in which the analyte/reagent interaction occurs. In certain sequencing-by-synthesis methods, for example, the template DNA is fragmented into lengths of several hundred base pairs in preparation for library generation. In certain embodiments, the isolation of the sample and the generation of the library are performed using automated methods and equipment.

In certain sequencing-by-synthesis procedures, the adapter oligonucleotides are complementary to the oligonucleotides present in the flow cells, and are sometimes used to associate the DNA with a solid support, the internal surface of a flow cell, for example. In some embodiments, the adapter oligonucleotide includes indexing oligonucleotides (indexes) (e.g. a unique nucleotide sequence usable as indexes to allow unambiguous identification of a sample), one or more primer sequencing hybridization sites (e.g. universal sequencing primers, single-end sequencing primers, paired-end sequencing primers, multiplexed sequence primers, and similar), or combinations thereof (e.g. adapter/sequencing, adapter/index, adapter/index/sequencing). Indexing primers are often six or more nucleotides in length. In certain embodiments, indexes are associated with a sample, but are sequenced in a separate sequencing reaction to avoid compromising the quality of the sequence reads. Subsequently, the sequence reads of the indexes and the sequence of the samples are linked together and the reads are demultiplexed.

In certain sequencing-by-synthesis methods, the use of oligonucleotide adapters allows the multiplexing of sequence reactions in a lane of a flow cell, which allows the analysis of multiple samples per lane of the flow cell. The number of samples that can be analysed in a given flow cell lane often depends on the number of unique indexes used during library preparation. The number of indexes used is not limited in the methods described in this document and such methods can be performed using any number of indexes (e.g. 4, 8, 12, 20, 24, 48 or 96). The greater the number of indexes, the greater the number of samples that can be multiplexed in a single flow cell lane. Multiplexing using 12 P7 indexes and 8 P5 indexes allows 96 samples to be analysed (e.g. equal to the number of wells in a 96-well plate) in a 2-lane flow cell.

In certain sequencing-by-synthesis procedures, modified template DNA is added with adapters to the flow cell and immobilized by hybridization with the anchor oligonucleotides present in the flow cell under conditions of limiting dilution. Multiple amplification cycles convert the DNA template of a single molecule into a clonally amplified cluster. For sequencing, the groups are denatured. Sequencing is initiated by hybridizing a primer complementary to the adapter sequences, followed by the addition of polymerase and a mixture of four reversible fluorescent probe terminators of different colours. The terminators are incorporated according to the sequence complementarity in each chain in a clonal cluster. After incorporation, the excess reagents are removed by washing, the groups are analysed optically and the fluorescence signal is recorded. With successive chemical steps, the terminators of reversible probes are unblocked, the fluorescent labels are cleaved and eliminated by washing, and the next sequencing cycle is performed.

**BRIEF DESCRIPTION OF THE FIGURES**

Figure 1. Profile of fragments of the indexed library. UF are fluorescence units.

Figure 2. Flow diagram of the bioinformatic analysis steps of the invention.

Figure 3. Distribution of Silhouette values for different histocompatibility genes.

Figure 4. Size profile obtained for libraries of samples VP346 (curve 3), VP347 (curve 2), VP348 (curves 4 and 5) and VP349 (curve 1). UF are fluorescence units.

Figure 5. Quality graph obtained for the sequence reads obtained with the combination of the libraries of samples VP346, VP347, VP348 and VP349 sequenced in Hiseq 2500 Rapid Mode® using a reading length of 100 bp. 93.8% of the sequence reads have a Q score greater than 30. A vertical line is shown at a Q value of 30.

Figure 6. Quality graph obtained for the sequence reads obtained with the combination of the libraries of samples VP346, VP347, VP348 and VP349 sequenced in Miseq® with a reading length of 250 bp. 89.4% of the sequence reads have a Q score greater than 30. A vertical line is shown at a Q score of 30.

Figure 7. Library size selection test with the libraries of samples VP347 and VP348. The results obtained with the TapeStation® 4200 equipment from Agilent® are shown. The results for an Ampure XP® beads ratio on the PCR result in the process of obtaining libraries of 0.7X from the library of sample VP347 (A7) and of sample VP348 (B7), the results for a Ampure XP® beads ratio on the PCR result in the process of obtaining libraries of 0.6X from the library of sample VP347 (C7) and of sample VP348 (D7) and the results for an Ampure XP® beads ratio on the result of PCR in the process of obtaining libraries of 0.5X of the library of sample VP347 (E7) and of sample VP348 (F7).

Figure 8. Quality graph obtained for the sequence reads obtained with the combination of the libraries tested in Figure 7 sequenced in MiSeq® with a reading length of 250 bp. 91.2% of the sequence reads have a Q score greater than 30. A vertical line is shown at a Q score of 30.

Figure 9. Statistical result of the contrast between the target sequence (corresponding to sample VP367) and family A*25 (A). It is observed that the target sequence is within the group of family A*25. The contrast performed by the Wilcoxon indicates that they are the same distributions by having a p value > = the cut-off value p $1^{-6}$. Phylogenetic tree of the target sequence with family A*25 (B)

Figure 10. Statistical result of the contrast between the target sequence (corresponding to sample VP367) and subfamily A*25:01 (A). It is observed that the target sequence is within the group of subfamily A*25:01. The contrast performed by the Wilcoxon indicates that they are the same distributions by having a pValue > = the cut-off value p $1^{-6}$. Phylogenetic tree of the target sequence with subfamily A*25:01 (B).

Figure 11. Statistical result of the contrast between the target sequence (corresponding to sample VP367) and subfamily A*25:01:01 (A). It is observed that the target sequence is within the group of subfamily A*25:01:01. The contrast performed by the Wilcoxon indicates that they are the same distributions by having a pValue > = the cut-off value p $1^{-6}$. Phylogenetic tree of the target sequence with subfamily A*25:01:01 (B).

Figure 12. Statistical result of the contrast between the target sequence (corresponding to sample VP367) and subfamily A*26. It is observed that the target sequence is outside the group of family A*26. The contrast of distributions indicates that it does not belong to the family with a pValue of $2^{-16}$, lower than the cut-off value p $1^{-6}$, therefore, according to the distances, the target sequence does not belong to family A*26.

Figure 13. Statistical result of the contrast between the target sequence (corresponding to sample VP371) and family C*12. It is observed that the target sequence is not within the group of family C*12. The contrast performed by the Wilcoxon indicates that they are not the same distributions by having a pValue of $2^{-16}$ lower than the cut-off value p $1^{-6}$.

Figure 14. Statistical result of the contrast between the target sequence (corresponding to sample VP371) and family C*12:03. It is observed that the target sequence is not within the group of the subfamily C*12:03. The contrast performed by the Wilcoxon indicates that they are not the same distributions by having a pValue of $2^{-16}$ lower than the cut-off value p $1^{-6}$.

## DESCRIPTION OF EMBODIMENTS

### Example 1. Analysed samples

Four (4) reference samples, provided by the international group called the International Histocompatibility Working Group were analysed: IHW09028, IHW01038, IHW09273 and IHW09014 corresponding to the samples VP346, VP347, VP348 and VP349, respectively. The HLA typing of all these samples were known before the test.

We also analysed clinical samples of real anonymised patients whose HLA typing was previously known before the trial (samples VP367, VP371 and VP373).

Genomic DNA samples were analysed. The genomic DNA was free of RNA and contaminants such as phenol, alcohol, EDTA or excess salts. The absorbance ratio 260/280 nm and 260/230 nm and the absorbance at 340 nm were checked.

### Example 2. Amplification by Long-Range PCR

Regions of interest from genomic DNA (gDNA) were amplified by long-range PCR. Subsequently, the amplification products were verified by electrophoresis and the amplicons were purified.

The long-range PCR reactions of the 11 amplicons of genes HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1 were prepared following the conditions described in Tables 2 and 3. Table 2 indicates whether the type of reaction mixture is "A" or "B" and Table 3 specifies the reaction mixtures for these two types of reaction mixture.

Table 2. Amplification conditions for the 11 amplicons

| Mixture of primers | Amplicon size | Type of reaction mixture | PCR program | ng of initial gDNA |
|---|---|---|---|---|
| I | 5478 | A | 1 | 100 |
| II | 6234 | A | 3 | 100 |
| III | 4536 | B | 1 | 100 |
| IV | 14267 | B | 3 | 100 |
| V | 12286 | B | 1 | 100 |
| VI | 11373 | B | 1 | 100 |
| VII | 9300 | A | 3 | 100 |
| VIII | 12437 | A | 3 | 100 |
| IX | 16360 | B | 3 | 100 |
| X | 15075 | B | 2 | 100 |
| XI | 13744 | A | 3 | 100 |

The 11 long-range PCR reactions were prepared on ice in tubes, using the volumes and amounts described in Table 3 and mixed.

Table 3. Reaction mixtures for long-range PCR

<table>
<tr><td rowspan="2">Reagents</td><td colspan="2">Volume for 1 reaction</td></tr>
<tr><td>Reaction mixture A</td><td>Reaction mixture B</td></tr>
<tr><td>Amplicon PCR Buffer 5X (PrimeSTAR® GXL DNA Polymerase (Takara Bio Inc).</td><td>10 μl</td><td>10 μl</td></tr>
<tr><td>dNTPs-AMP</td><td>4 μl</td><td>4 μl</td></tr>
<tr><td>DNA Polymerase</td><td>1 μl</td><td>1 μl</td></tr>
<tr><td>I-XI (20 μM)</td><td>0.5 μl</td><td>0.5 μl</td></tr>
<tr><td>Betaine</td><td>-</td><td>13 μl</td></tr>
<tr><td>gDNA</td><td>100 ng</td><td>100 ng</td></tr>
<tr><td>Nuclease-free water</td><td>Up to 50 μl</td><td>Up to 50 μl</td></tr>
</table>

The tubes were placed in the thermocycler and the programs indicated in Table 4 for each amplicon were executed.

Table 4. Program for the amplification of the amplicons

<table>
<tr><td colspan="5">Program for the amplification of the amplicons I, III, V, VI and IX</td></tr>
<tr><td>Program</td><td>Step</td><td>No. Cycles</td><td>Temperature</td><td>Time</td></tr>
<tr><td rowspan="3">3</td><td rowspan="2">1</td><td rowspan="2">30</td><td>98 °C</td><td>10 s</td></tr>
<tr><td>68 °C</td><td>10 min</td></tr>
<tr><td>2</td><td>1</td><td>4 °C</td><td>Hold</td></tr>
<tr><td colspan="5">Program for the amplification of the amplicon X</td></tr>
<tr><td>Program</td><td>Step</td><td>No. Cycles</td><td>Temperature</td><td>Time</td></tr>
<tr><td rowspan="3">3</td><td rowspan="3">1</td><td rowspan="3">30</td><td>98 °C</td><td>10 s</td></tr>
<tr><td>60 °C</td><td>15 s</td></tr>
<tr><td>68 °C</td><td>10 min</td></tr>
<tr><td></td><td>2</td><td>1</td><td>4 °C</td><td>Hold</td></tr>
<tr><td colspan="5">Program for the amplification of the amplicons II, IV, VII and XI</td></tr>
<tr><td>Program</td><td>Step</td><td>No. Cycles</td><td>Temperature</td><td>Time</td></tr>
<tr><td rowspan="3">3</td><td rowspan="3">1</td><td rowspan="3">30</td><td>98 °C</td><td>10 s</td></tr>
<tr><td>55 °C</td><td>15 s</td></tr>
<tr><td>68 °C</td><td>10 min</td></tr>
<tr><td></td><td>2</td><td>1</td><td>4 °C</td><td>Hold</td></tr>
</table>

When the thermocycler program ended, the samples were left on ice.

**Control of amplification and purification of the amplicons**

The amplification products were checked by electrophoresis on a 0.8% agarose gel. To perform the electrophoresis, molecular weight markers Low DNA Mass Ladder (Invitrogen) Low-Mass and Lambda DNA/HindIII Marker (Thermo Fisher) were used, and a BPB (Bromophenol Blue sodium salt (Sigma-Aldrich)) loading buffer was used. Each of the amplicons was purified independently. The result of the purification was checked again by agarose gel electrophoresis and it was found that the primers in the sample had been completely removed. The concentration of each of the amplicons was quantified.

**Example 3. Combination of amplicons**

An equimolar combination of 1 μg per sample was prepared. Depending on the size of each amplicon, a certain amount of each amplification purification was used, as indicated in Table 5. The combination was prepared with the amplicons of each of the samples independently.

Table 5. Preparation of an equimolar combination of purified amplicons

| Amplicon | Size (bp) | ng for 1 μg of equimolar pool |
|---|---|---|
| I | 5478 | 45.2 |
| II | 6234 | 51.5 |
| III | 4536 | 37.5 |
| IV | 14267 | 117.8 |
| V | 12286 | 101.5 |
| VI | 11373 | 93.9 |
| VII | 9300 | 76.8 |
| VIII | 12437 | 102.7 |
| IX | 16360 | 135.1 |
| X | 15075 | 124.5 |
| XI | 13744 | 113.5 |
| Total | 121090 | 1000 |

Electrophoresis was used to check that there were no traces of primers in the final product and the final product was purified by Zymo-Spin® IC-XL (Zymo Research) columns according to the manufacturer's instructions for purification of PCR products. The absorbance ratio 260/280 nm and 260/230 nm and the absorbance at 340 nm were checked. The final concentration of the combination was quantified.

**Example 4. Tagmentation of amplicons**

The amplified DNA was enzymatically cleaved with the enzyme tagmentase (a kind of transposase), which randomly fragments the DNA into fragments of the desired size by incubation at 45 °C for 10 minutes. Simultaneously, the enzyme adds specific sequences at the ends of the fragmented DNA that will allow the incorporation of the adapters in the following PCR amplification process. This process is known as tagmentation, which results in tagmented DNA. Subsequently, the tagmented DNA was purified using Agencourt AMPure XP® magnetic beads (Beckman Coulter).

The fragmentation reactions were prepared in ice using tubes. 17 μl of TB (GeneSGKit® Tagmentation Buffer (Sistemas Genómicos)) buffer were added to each tube. 2 μl of amplified DNA were added to its corresponding tube, with the tip of the pipette into the bottom of the tube. 0.5 μl TE buffer (GeneSGKit® Tagmentation Enzyme (Sistemas Genómicos)) were added to each tube, with the tip of the pipette at the bottom of the tube and mixed. The tubes were placed in the thermocycler and the thermocycler program of Table 6 was followed.

Table 6. Thermocycler program for DNA fragmentation.

| Step | Temperature | Time |
|---|---|---|
| 1 | 45°C | 10 minutes |
| 2 | 4C° | 1 minute |
| 3 | 4C° | *Hold* |

The samples were incubated for 1 minute at 4 °C in the thermocycler and transferred to ice. 32 μl of SS (GeneSGKit® Stop Solution (Sistemas Genómicos)) buffer were added to each reaction and mixed. 52 μl of the Agencourt

AMPure XP® beads (Beckman Coulter) solution was added to each tube containing the sample and mixed. The tubes were placed into the magnetic holder and left for 5 minutes. With the tubes in the magnetic holder, the supernatant was removed with a pipette, avoiding touching the Agencourt AMPure XP® magnetic beads (Beckman Coulter). The tubes were washed 2 times with 200 μl of 70% ethanol and all the ethanol removed with a pipette. Tubes were kept in the magnetic holder and were left to dry with the cap open at room temperature for 5 minutes or until all residual ethanol had evaporated. 11 μl of nuclease-free water were added to each tube, mixed and incubated at room temperature for 2 minutes. The tubes were placed into the magnetic holder for 2-3 minutes, until the solution did not contain Agencourt AMPure XP® (Beckman Coulter) magnetic beads. The supernatant (~10 μl) was transferred to a new 0.2 ml tube and the Agencourt AMPure XP® (Beckman Coulter) magnetic beads were removed.

**Example 5.1. Amplification and indexing of the libraries. Combination of the libraries**

In this step, the tagmented and purified DNA was amplified by a small number of PCR cycles. All the tagmented DNA was used. After amplification, the library was purified using magnetic beads.

A sequence of specific nucleotides (index) was added to each individual library by PCR amplification, enabling mixing of various libraries to sequence them together in a single lane. The lane is the basic physical unit in a Next Generation sequencing machine and refers to the basic independent execution in a Next Generation sequencing machine.

Libraries were mixed so that each had a single P7 index. With this combination strategy, only the sequencing of an index (P7) was necessary. The combinations of Table 7 were used. Each position produced a fluorescence signal in the 2 channels during the sequencing of the index. The nucleotide sequence for the indexes is shown in Table 8.

**Example 5.2. Amplification and indexing of the libraries. Combination of the libraries**

Libraries were mixed so that the P7 indexes were not unique. In this case, different P5 indexes were included. The combinations of Table 7 were used. Each position produced a fluorescence signal in the 2 channels during the sequencing of the index. The nucleotide sequence for the indexes is shown in Table 8.

Table 7. Combinations of libraries

<table>
<tr><th>Complexity of the pool</th><th>P7 indexes used</th><th>P5 indexes used</th><th>Sequencing</th></tr>
<tr><td>1-plex (without pooling)</td><td>Any P7 index (1-12)</td><td rowspan="6">Any P5 index (13-20)</td><td>Without sequencing of the index</td></tr>
<tr><td>2-plex</td><td>Combination A: 1 and 2<br>Combination B: 2 and 4</td><td rowspan="5">Sequencing of a single index (P7)</td></tr>
<tr><td>3-plex</td><td>Combination A: 1, 2 and 4<br>Combination B: 3, 12 and 6</td></tr>
<tr><td>4- or 5-plex</td><td>Combination A: 1, 2 and 4 and any other P7 index<br>Combination B: 3, 12 and 6 and any other P7 index</td></tr>
<tr><td>6-plex</td><td>1, 2, 3, 4, 6 and 12</td></tr>
<tr><td>7- to 12-plex</td><td>1, 2, 3, 4, 6, 12 and any other P7 index</td></tr>
<tr><td>> 12-plex</td><td>1,2,3,4,6,12 and any other P7 index</td><td>Combination A: 13, 14 and any other P5 index<br>Combination B: 15, 16 and any other P5 index<br>Combination C: 17, 18 and any other P5 index</td><td>Sequencing of 2 indexes (P7 and P5)</td></tr>
</table>

Table 8. Sequencing of nucleotides of P7 and P5 indexes

| Index P7 | Sequence | Index P5 | Sequence |
|---|---|---|---|
| 1 | TAAGGCGA | 13 | TAGATCGC |
| 2 | CGTACTAG | 14 | CTCTCTAT |
| 3 | AGGCAGAA | 15 | TATCCTCT |
| 4 | TCCTGAGC | 16 | AGAGTAGA |
| 5 | GTAGAGGA | 17 | GTAAGGAG |
| 6 | TAGGCATG | 18 | ACTGCATA |
| 7 | CTCTCTAC | 19 | AAGGAGTA |
| 8 | CAGAGAGG | 20 | CTAAGCCT |
| 9 | GCTACGCT | | |
| 10 | CGAGGCTG | | |
| 11 | AAGAGGCA | | |
| 12 | GGACTCCT | | |

The combinations described in Table 9 were used.

Table 9. Combinations of indexes used

| Index 1 | Index 2 |
|---|---|
| TAAGGCGA | TAGATCGC |
| TCCTGAGC | CTCTCTAT |
| GTAGAGGA | TAGATCGC |
| TAGGCATG | CTCTCTAT |
| CGTACTAG | CTCTCTAT |
| AGGCAGAA | TAGATCGC |
| CTCTCTAC | TAGATCGC |
| CAGAGAGG | CTCTCTAT |
| TAAGGCGA | CTCTCTAT |

The volume required of PCR Master Mix (PCRMM) was prepared in ice as described in Table 10 and mixed.

Table 10. PCR Master Mix for the amplification of tagmented DNA

| Reagents | Volume for 1 reaction | Volume for 12 reactions |
|---|---|---|
| Nuclease-free water | 22 μl | 277.2 μl |
| GeneSGKit® 5X PCR Buffer | 10 μl | 126 μl |
| GeneSGKit® dNTPs | 0.5 μl | 6.3 μl |
| DMSO | 2.5 μl | 31.5 μl |
| GeneSGKit® PCR Enzyme | 1 μl | 12.6 μl |
| Total | 36 μl | 453.6 μl |

36 μl of PCRMM were added to each tube containing 10 μl of tagmented and purified DNA obtained in the previous steps. 2 μl of one of the primers GeneSGKit® P7 indexing primers (1-12) were added to each PCR mixture. 2 μl of one of the primers GeneSGKit® P5 indexing primers (13-20) were added to each PCR mixture and mixed. The tubes were placed in the thermocycler and the program of Table 11 was executed.

Table 11. Thermocycler program for amplifying the tagmented DNA

| Step | No. cycles | Temperature | Time |
|---|---|---|---|
| 1 | 1 | 68°C | 2 minutes |
| 2 | 1 | 98°C | 30 seconds |
| 3 | 5 | 98°C | 30 seconds |
| | | 56°C | 30 seconds |
| | | 72°C | 1 minute |
| 4 | 1 | 4°C | *Hold* |

The solution of Agencourt AMPure XP® (Beckman Coulter) magnetic beads was incubated at room temperature for 30 minutes. The Agencourt AMPure XP® (Beckman Coulter) magnetic beads were vigorously mixed until a homogeneous suspension was obtained. 30 μl of the Agencourt AMPure XP® (Beckman Coulter) beads solution were added to each tube containing the sample, mixed and incubated for 5 minutes at room temperature. The tubes were placed into the magnetic holder and left for 5 minutes. The tubes were kept in the magnetic holder while the supernatant was removed with a pipette, avoiding touching the beads. With the tubes in the magnetic holder, they were washed 2 times adding 200 μl of 70% ethanol to each tube. The tubes were left in the magnetic holder for 1 minute and the ethanol was removed with the help of a pipette. The tubes were kept in the magnetic holder and dried with the cap open at room temperature for 5 minutes. 30 μl of nuclease-free water were added to each tube, mixed and incubated at room temperature for 2 minutes. The tubes were placed in the magnetic holder for 2-3 minutes, until the solution was clear, without beads. The supernatant (~ 30 μl) was transferred to a new tube and the Agencourt AMPure XP® (Beckman Coulter) magnetic beads were removed.

**Example 6. Validation of the libraries**

The quality of the library obtained was determined by analysing an aliquot of the library on an Agilent 2100 Bioanalyzer Platform® (Agilent), using the Agilent High Sensitivity DNA assay® (Agilent) kit following instructions and protocols of the manufacturer to carry out this analysis.

An electropherogram was obtained that shows a DNA peak between 1200-1500 bp, shown in Figure 1.

**Example 7. Combination of libraries for sequencing**

The concentration of each library was calculated. The amount of each of the libraries that should be used for mixing and making the pool was determined using the following formula:

$$\text{Volume of library} = (Vf \times Cf)/(nl \times Ci)$$

wherein

Vf is the final volume of the pool

Cf is the final concentration of the library in the pool (4 nM)

nl is the number of libraries

Ci is the initial concentration of each library (nM)

The libraries were combined using the volume determined with the above formula. The final volume of the library pool was adjusted to the final concentration with 10 mM Tris-Cl, pH 8.5, 0.1% v/v Tween 20.

**Example 8. Sequencing of the libraries by Next Generation sequencing**

The concentration of the sequenced library or pool of libraries was 17 pM. The libraries were sequenced on the Next Generation sequencing platforms MiSeq® or HiSeq® 2500 from Illumina.

A number of primers, GeneSGKit® Read Primer 1 (RP1) (Sistemas Genómicas), GeneSGKit® Index Read Primer (iRP) (Sistemas Genómicos) and GeneSGKit® Read Primer 2 (RP2) were added to the Next Generation sequencing platforms MiSeq® from Illumina to the cartridge of the sequencing platform. The cartridge also contained Illumina *Sequencing Primers*® (Illumina, Inc.). The amount of primers added is shown in Table 12.

Table 12. Preparation of sequencing primers

| Reagent | Well | Amount added |
|---|---|---|
| GeneSGKit® Read Primer 1 | 12 | 3.4 μl |
| GeneSGKit® Index Read Primer | 13 | 3.4 μl |
| GeneSGKit® Read Primer 2 | 14 | 3.4 μl |

In sequencing on Next Generation sequencing platforms for HiSeq® 2000 y HiSeq® 2500 from Illumina in High Output mode, the primers RP1, iRP and RP2 were combined with the Illumina Sequencing Primers® from Illumina indicated in Table 13 and in the amounts shown in this table.

Table 13. Preparation of sequencing primers

| Reagent | Volume of GeneSGKit® primer | Volume of Illumina® Primer |
|---|---|---|
| GeneSGKit® Read Primer 1 | 5 μl | 995 μl HP6 or HP10 |
| GeneSGKit® Index Read Primer | 15 μl | 2985 μl HP8 or HP12 |
| GeneSGKit® Read Primer 2 | 15 μl | 2985 μl HP7 or HP11 |

In sequencing on the Next Generation sequencing platforms HiSeq® 2500 de Illumina in Rapid Mode, the primers RP1, iRP and RP2 were combined with the Illumina Sequencing Primers from Illumina shown in Table 14 and in the amounts shown in this table.

Table 14: Preparation of sequencing primers

| Reagent | Abbreviation | Volume of GeneSGKit® primer | Volume of Illumina® Primer |
|---|---|---|---|
| GeneSGKit® Read Primer 1 | RP1 | 8.8 μl | 1741.2 μl HP10 |
| GeneSGKit® Index Read Primer | iRP | 8.8 μl | 1741.2 μl HP12 |
| GeneSGKit® Read Primer 2 | RP2 | 8.8 μl | 1741.2 μl HP11 |

Primers were added into the corresponding cartridge well with a pipette tip (or Pasteur pipette). The primers were mixed well avoiding the formation of bubbles. The cartridge was observed to ensure that there were no bubbles. Bubbles were removed by gently tapping the cartridge on the table.

The lane was then prepared on the Next Generation sequencing platform MiSeq® or HiSeq® from Illumina, following the manufacturer's instructions.

**Example 9. Identification of each sequence**

Figure 2 shows a flow diagram of the stages of the bioinformatic analysis, described in detail below.

**Example 9.1. Determination of raw data quality**

Massive sequencing (NGS, Next generation Sequencing) generated values of probability of error per base, called quality values. These quality values were presented on the Phred scale, where a value of 20 means that there is a probability of 1/100 that the base was the product of an error. The overall study of the data quality provided information on the sequencing quality.

**Example 9.2. Sequence alignment and evaluation of the design of the enrichment of**

**capture**

The reads obtained were aligned against the reference genome version GRCh38/hg38 by BWA (mem algorithm). After the mapping of the reads, those sequences that introduced greater biases and noise that would affect the following analysis steps were filtered. From the formatted BAM file retrieved after filtering, low-quality sequences and those labelled as PCR duplicates were removed. In addition, the global coverage of the sample and the efficiency of the designed strategy were evaluated at this point.

**Example 9.3. Variant calling**

Variants were identified by variant calling. This identification was performed by the analysis of the results of the reads from the sequencing. The mismatches identified between the obtained reading and the reference genome were studied in greater depth in order to identify real variants of the sample. The Broad Institute (https://software.broadinstitute.org/gatk/) GATK (Genome Analysis Tool_Kit) algorithm was used for this phase (McKenna, A. et al. (2010)).

**Example 9.4. Determination of haplotype blocks and amplification of the limits of these blocks**

The identification of haplotype blocks implies the determination of fragments of the sequences of the alleles to be determined that have specific variants and that are as long as possible, thus distinguishing between the different sequences of each of the alleles in case of being heterozygous or, in the case of homozygosis, a single sequence with its variants with respect to the reference. The determination of haplotype blocks is based on the alignment of the reads against the reference genes and the identification of previously determined variants on this sequence.

The high overlap of the reads aligned against the reference genome (due to the great depth of the reading used in the analysis) as well as the large size of the reading length used (2 x 250 nucleotides) and the size of the insert enabled discrimination, using the WhatsHap software (https://whatshap.readthedocs.io/en/latest/) (Patterson, M. et al. (2015)) of haplotype blocks that in many cases covered the complete studied gene. The sequence of these blocks was obtained with the bcftools tool (https://www.sanaer.ac.uk/science/tools/samtools-bcftools-htslib). When these sequences were obtained, the first process of identification of the alleles was started.

In addition, it was often the case that a specific haplotype block was flanked by homozygous regions that were not recorded in said block, the addition of these homozygous flanking regions facilitated the process of identification of the allele because increasing the size of the sequence reduces ambiguity.

**Example 9.5. Local alignment of the sequence of haplotype blocks obtained against all the alleles of IPD-IMGT/HLA for each gene**

The first step of identification of the alleles was performed by local alignment of the sequences obtained from the haplotype blocks in each case against all the genomic sequences of all the alleles present in the IPD-IMGT/HLA database (https://www.ebi.ac.uk/ipd/imgt/hla/) for each gene. This was performed with the fasta36 program (Pearson, W. R. (2016)).

The output from this operation provided a list of possible candidates to the allele to be identified. The output of this process prioritised according to the percentage of sequence identity with the allele in question is shown in Tables 15A and 15B.

Tables 15A and 15B. Ten primary candidates to be the correct haplotype of sample VP347 prioritised by the percentage similarity of the sequence

Table 15A

| Allele | % identity | Alignment length | Mismatches | Deletion/insertion (gap opens) | Start of target sequence |
|---|---|---|---|---|---|
| A*32:04 | 99.80 | 3503 | 5 | 2 | 785 |
| A*03:01:01:01 | 99.80 | 3503 | 5 | 2 | 785 |

(continued)

| Allele | % identity | Alignment length | Mismatches | Deletion/insertion (gap opens) | Start of target sequence |
|---|---|---|---|---|---|
| A*03:284N | 99.77 | 3503 | 5 | 3 | 785 |
| A*03:272 | 99.77 | 3503 | 6 | 2 | 785 |
| A*03:271 | 99.77 | 3503 | 6 | 2 | 785 |
| A*03:269N | 99.77 | 3503 | 6 | 2 | 785 |
| A*03:268 | 99.77 | 3503 | 6 | 2 | 785 |
| A*03:267 | 99.77 | 3503 | 6 | 2 | 785 |
| A*03:265 | 99.77 | 3503 | 6 | 2 | 785 |
| A*03:264 | 99.77 | 3503 | 6 | 2 | 785 |

Table 15B

| Allele | Target final sequence | Start of destination sequence | End of destination sequence | e value | Bit score value |
|---|---|---|---|---|---|
| A*32:04 | 4286 | 1 | 3502 | 0 | 4707.8 |
| A*03:01:01:01 | 4286 | 1 | 3502 | 0 | 4707.8 |
| A*03:284N | 4286 | 1 | 3501 | 0 | 4702.1 |
| A*03:272 | 4286 | 1 | 3502 | 0 | 4705.3 |
| A*03:271 | 4286 | 1 | 3502 | 0 | 4705.3 |
| A*03:269N | 4286 | 1 | 3502 | 0 | 4705.3 |
| A*03:268 | 4286 | 1 | 3502 | 0 | 4705.3 |
| A*03:267 | 4286 | 1 | 3502 | 0 | 4705.3 |
| A*03:265 | 4286 | 1 | 3502 | 0 | 4705.3 |
| A*03:264 | 4286 | 1 | 3502 | 0 | 4705.3 |

The e value is a mathematical parameter that indicates the number of bases within the sequence with respect to those expected to be modified by chance.

The value bit score is a logarithmic scale value. The score is a mathematical value that indicates the quality of the alignment based on several parameters, including length of the sequence, array of substitution and value of penalty.

In Tables 15A and 15B, the first two candidates have identical output parameters and are therefore indistinguishable. This poses a problem because there is a single solution. To solve this problem, a second classification criterion was performed based on the phylogenetic distances of the candidates to the families of all the possible alleles of each gene.

**Example 9.6. Pre-calculation of distances from the IPD-IMGT/HLA database**

Various previous studies evaluated the diversity of the histocompatibility sequences from the evolutionary and hierarchical points of view (Gu, X. et al. (1999); McKenzie, L. M. et al. (1999)). These studies were able to identify groups of alleles, belonging to different HLA sequences, describing a phylogenetic relationship and establishing a relationship with the serological behaviour.

The quality of the phylogenetic grouping of the various families was assessed, at different levels, of HLA contained in the IPD-IMGT/HLA database (https://www.ebi.ac.uk/ipd/imgt/hla/). To do this, the multiple alignments of the families were taken using the MAFFT method (Katoh, K. et al. (2013)). The genetic distances and their evolutionary relationship were calculated using the neighbour-joining method (Saitou, N. et al. (1987)). The final result was an NxN matrix, wherein the phylogenetic distances of all the sequences compared to all others are established and where N is the number of alleles in the reference HLA database of alleles (e.g. the IPD-IMGT/HLA database (https://www.ebi.ac.uk/ipd/imgt/hla/)). The number of alleles in the HLA alleles database can vary with time. The distance matrix is a matrix that contains the distance values of each allele with itself and with the others.

The quality of the families was assessed from the phylogenetic point of view using the silhouette method (Rousseeuw, P. J. (1987)). This method is a cluster evaluator. This methodology enabled evaluating if the phylogenetic distances or relationships between members of a same family are statistically closer compared to the other families in the database. The silhouette value varies between -1 and 1; the closer it is to 1, the more defined is the group. Normally, values above 0.5 are considered acceptable groups.

The results obtained for the IPD-IMGT/HLA database (https://www.ebi.ac.uk/ipd/imgt/hla/), version 3.32, are shown in Figure 3. Different families of histocompatibility genes, at different levels, have silhouette values close to 1, indicating a phylogenetic closeness compared to the other sequences.

**Example 9.7. Primary selection of candidate alleles**

In a first step, for an overall selection of possible HLA candidates, a contrast of overall homology was performed using the FASTA36 algorithm (Pearson, W. R. (2016)). The global contrast enabled evaluating the proximity of the identified haplotype(s) using the whole length as a unit, avoiding similarity by minor domains or profiles. The selection of a small group of possible HLAs was based on a combination of parameters, such as those that define the homology, the percentage of identity and the length of the alignment.

**Example 9.8. Phylogenetic study**

Once the subgroup of possible HLA candidates with better global homology parameters was identified, an evolutionary phylogenetic study was performed. This phylogenetic study was performed using the R platform and phangorn library (Schliep K. P. (2011); Schliep, K. P. et al. (2017)). We started with the genetic DNA sequences of the pre-selected HLA and the haplotype or haplotypes associated with the target samples. The genetic distance was calculated in the context of "all against all" using the F81 strategy (Li, H. et al. (2009)). Subsequently, the phylogenetic tree was generated using the neighbour-joining strategy (Felsenstein, J. (1981); Saitou, N. et al. (1987); Studier, J. A. et al. (1988)). Finally, the distances matrix was accessed using the cophenetic method (Gascuel, O. (1997)).

**Example 9.9. Contrast test**

The contrast test started with the phylogenetically closest HLA allele obtained from the IPD-IMGT/HLA (https://www.ebi.ac.uk/ipd/imgt/hla/) database and obtained from the phylogenetic study (called contrasted HLA) and the target haplotype. The assignment of the family and the various levels of the target haplotype were equalled to the contrasted HLA. Subsequently, the sequence of the contrasted HLA was traversed from the upper to the lower level. At each level, the mean, median and the confidence intervals of each distribution distance of the family members at an identical level, obtained in Example 9.4, were evaluated. If the distance between the target haplotype and the closest contrasted HLA is within the theoretical distribution of the level studied with a significance of 95%, it was considered to belong to that level in a statistically significant way (Sneath, P. H. A. et al. (1973); Bauer, D. F. (1972)). The test ended with a statistical identification of the target haplotype with the different levels. The target haplotype was considered to be the contrasted allele if the 3 higher levels were significant. If this premise was not met, the process was repeated with the second highest homology HLA, and thus recursively until all the HLA obtained in Example 9.5 were completed.

**Example 9.10. Assignment of the levels and families of the target haplotype**

Each target haplotype obtained in Example 9.9 had statistically significant levels. The target haplotypes that did not obtain this significance were assigned as "de novo" alleles in the corresponding level.

**Example 10. Analysis of the results**

The generated libraries of the 4 reference samples VP346, VP347, VP348 and VP349 were analysed.

Tagmentation and sequencing were performed using the Agilent SureSelectQXT Whole Genome Library Prep kit from Illumina Multiplexed Sequencing. The approximate size of the insert was 400 bp. The profile of sizes obtained from the VP346, VP347, VP348 and VP349 libraries is shown in Figure 4. The 4 libraries were sequenced on the Hiseq® 2500 sequencing platform from Illumina in Rapid Mode using a reading length of 100 bp. The quality graph is shown in Figure 5.

Subsequently, the same libraries were sequenced on Miseq® with a reading length of 250 bp. The quality graph is shown in Figure 6.

Tagmentation tests were performed on the samples VP347 and VP348. A library size selection test was also performed using the ratios 0.7X, 0.6X and 0.5X of Ampure XP beads® on the result of the PCR in the process of obtaining the libraries. The results obtained are shown in Figure 7.

The 6 libraries were sequenced on MiSeq® with a reading length of 250 bp. The quality graph is shown in Figure 8.

**Sample VP367**

The final results of the comparison of sample VP367 for the allele HLA*A are shown in Tables 16A and 16B.

Tables 16A and 16B. Comparison of sample VP367 for the allele HLA*A

Table 16A

| Allele | % identity | Alignment length | Mismatches | Deletion/insertion (gap opens) | Start of target sequence |
|---|---|---|---|---|---|
| A*25:01:01:01 | 99.49 | 3517 | 1 | 17 | 785 |
| A*25:42N | 99.46 | 3517 | 2 | 17 | 785 |
| A*25:41 | 99.46 | 3517 | 2 | 17 | 785 |
| A*25:40 | 99.46 | 3517 | 2 | 17 | 785 |
| A*25:39 | 99.46 | 3517 | 2 | 17 | 785 |
| A*25:38 | 99.46 | 3517 | 2 | 17 | 785 |
| A*25:29 | 99.46 | 3517 | 2 | 17 | 785 |
| A*25:02 | 99.46 | 3517 | 2 | 17 | 785 |
| A*25:01:11 | 99.46 | 3517 | 2 | 17 | 785 |
| A*25:01:10 | 99.46 | 3517 | 2 | 17 | 785 |
| A*25:27:02 | 99.43 | 3517 | 3 | 17 | 785 |
| A*25:44 | 99.37 | 3517 | 5 | 17 | 785 |
| A*25:43 | 99.35 | 3517 | 6 | 17 | 785 |
| A*26:01:01:01 | 99.26 | 3517 | 9 | 17 | 785 |
| A*26:17 | 99.23 | 3517 | 10 | 17 | 785 |
| A*26:148 | 99.23 | 3517 | 10 | 17 | 785 |
| A*26:137 | 99.23 | 3517 | 10 | 17 | 785 |

Table 16B

| Allele | Target final sequence | Start of destination sequence | End of destination sequence | e value | Bit score value |
|---|---|---|---|---|---|
| A*25:01:01:01 | 4284 | 1 | 3517 | 0 | 8846.2 |
| A*25:42N | 4284 | 1 | 3517 | 0 | 8841.6 |
| A*25:41 | 4284 | 1 | 3517 | 0 | 8841.6 |
| A*25:40 | 4284 | 1 | 3517 | 0 | 8841.6 |

(continued)

| Allele | Target final sequence | Start of destination sequence | End of destination sequence | e value | Bit score value |
|---|---|---|---|---|---|
| A*25:39 | 4284 | 1 | 3517 | 0 | 8841.6 |
| A*25:38 | 4284 | 1 | 3517 | 0 | 8841.6 |
| A*25:29 | 4284 | 1 | 3517 | 0 | 8841.6 |
| A*25:02 | 4284 | 1 | 3517 | 0 | 8841.6 |
| A*25:01:11 | 4284 | 1 | 3517 | 0 | 8841.6 |
| A*25:01:10 | 4284 | 1 | 3517 | 0 | 8841.6 |
| A*25:27:02 | 4284 | 1 | 3517 | 0 | 8837.0 |
| A*25:44 | 4284 | 1 | 3517 | 0 | 8827.8 |
| A*25:43 | 4284 | 1 | 3517 | 0 | 8823.2 |
| A*26:01:01:01 | 4284 | 1 | 3517 | 0 | 8809.4 |
| A*26:17 | 4284 | 1 | 3517 | 0 | 8804.8 |
| A*26:148 | 4284 | 1 | 3517 | 0 | 8804.8 |
| A*26:137 | 4284 | 1 | 3517 | 0 | 8804.8 |

Tables 16A and 16B show that there is one candidate, the HLA sequence A*25:01:01:01 that has better similarity statistics, which a priori can indicate that it is the most similar. The methods of the state of the art only offer this result, the HLA sequence that is most similar to the target sequence. In the previous examples of the invention, it was determined that the families of the HLA*A genes at different levels have a good grouping or phylogenetic clusters. Next, it was determined if the distribution of phylogenetic distances of the target sequence with the A*25 family and the A*25 family were only statistically identical or compatible. If the distribution of phylogenetic distances of the target sequence with the A*25 family and the A*25 family are only statistically identical or compatible, the method of the invention determines that the target sequence belongs to the A*25 family and goes to the next level, in this case, A*25:01. The statistical results for the A*25 can be seen in Figure 9, for the subfamily A*25:01 in Figure 10 and for the subfamily A*25:01:01 in Figure 11.

The method of this invention indicates not only the closest sequence, as the methods of the state of the art, but, with the contrast of phylogenetic distributions between the family and the various subfamilies with the target sequence, the method determines, statistically, that it belongs to the group A*25:01:01.

The specificity of the method was assessed by performing the same calculation for the next closest family, family A*26. The results are shown in Figure 12.

**Sample VP371**

The final results of the comparison of the sample VP371 for the HLA*C allele are shown in tables 17A and 17B.

Tables 17A and 17B. Comparison of sample VP367 for the HLA*C allele

Table 17A

| Allele | % identity | Alignment length | Mismatches | Deletion/insertion (gap opens) |
|---|---|---|---|---|
| C*12:03:01:01 | 99.77 | 4303 | 10 | 0 |
| C*12:20 | 99.72 | 4303 | 12 | 0 |
| C*12:18:01 | 99.67 | 4303 | 14 | 0 |
| C*06:02:01:02 | 99.51 | 4303 | 21 | 0 |

(continued)

| Allele | % identity | Alignment length | Mismatches | Deletion/insertion (gap opens) |
|---|---|---|---|---|
| C*06:02:01:01 | 99.51 | 4303 | 21 | 0 |
| C*06:58 | 99.49 | 4303 | 22 | 0 |
| C*06:07 | 99.49 | 4303 | 22 | 0 |
| C*06:06 | 99.49 | 4303 | 22 | 0 |
| C*06:02:20 | 99.49 | 4303 | 22 | 0 |
| C*06:02:01:12 | 99.49 | 4303 | 22 | 0 |

Table 17B

| Allele | Start of target sequence | Target final sequence | Sense |
|---|---|---|---|
| C*12:03:01:01 | 4808 | 506 | antisense |
| C*12:20 | 4808 | 506 | antisense |
| C*12:18:01 | 4808 | 506 | antisense |
| C*06:02:01:02 | 4808 | 506 | antisense |
| C*06:02:01:01 | 4808 | 506 | antisense |
| C*06:58 | 4808 | 506 | antisense |
| C*06:07 | 4808 | 506 | antisense |
| C*06:06 | 4808 | 506 | antisense |
| C*06:02:20 | 4808 | 506 | antisense |
| C*06:02:01:12 | 4808 | 506 | antisense |

Tables 17A and 17B show that there is one candidate, the HLA sequence C*12:01:01:01, that has better similarity statistics, which a priori can indicate that it is the most similar. The methods of the state of the art only offer this result, the HLA sequence that is most similar to the target sequence. In the previous examples of the invention, it was determined that the families of the HLA*C genes at different levels have a good grouping or phylogenetic clusters. Next, it was determined if the distribution of phylogenetic distances of the target sequence with the C*12 family and the C*12 family were only statistically identical or compatible.

The example of contrast between the target sequence and the C*12 family is shown in Figure 13.

This example shows that despite the target sequence more resembling the C*12 family, as indicated by the methods of the state of the art, the method of the invention determines that the divergence of the sequence is statistically greater than the endogenous divergence of the C*12 family. This divergence indicates that it does not belong to this family, so the target sequence is a *de novo* sequence.

This conclusion is retained in the C*12:03 subfamily, as shown in Figure 14.

**Example 10.1. Selection of a significant cut-off value**

A cut-off value p of $1^{-6}$ was selected, so that a target sequence is identified as a sequence that encodes for at least one locus of known HLA when a value of p obtained in the contrast test is greater than or equal to the cut-off value of $1^{-6}$ or, alternatively as a de *novo* sequence that encodes for a new HLA locus when a value of p obtained in the contrast test that is less than this cut-off value.

**Example 11. Visualisation of the results**

A results report was created for visualisation of the results of the method of genotypic identification of HLA of the invention. The report of results generated for the sample VP373 are shown in Table 18 as an example.

Table 18. HLA results report

<table>
<tr><td colspan="6">HLA RESULTS REPORT</td></tr>
<tr><td colspan="6">Sex: Male<br>Sample No.: KT-00000013_08_VP373<br>External Ref.: VP373</td></tr>
<tr><td colspan="6">1. Pathology of origin<br>SCID T- B-</td></tr>
<tr><td colspan="6">2. Clinical study indication<br>Patient with immunophenotype indicative of IDCS and repeated viral and bacterial repetition</td></tr>
<tr><td colspan="6">3. Results</td></tr>
<tr><td>Gene</td><td></td><td colspan="2">Genetic allele</td><td colspan="2">Serological allele</td></tr>
<tr><td rowspan="20">HLA_A</td><td rowspan="2">Major contig alleles</td><td>A*24:02:01:01</td><td>A*02:01:01:05</td><td rowspan="2">A*24</td><td rowspan="2">A*02</td></tr>
<tr><td>100.0%</td><td>99.46%</td></tr>
<tr><td rowspan="2">Minor contig alleles</td><td>A*24:68</td><td>A*02:01:01:01</td><td rowspan="2">A*24</td><td rowspan="2">A*02</td></tr>
<tr><td>99.97%</td><td>99.46%</td></tr>
<tr><td rowspan="2">Minor contig alleles</td><td>A*24:56</td><td>A*02:90</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:53</td><td>A*02:704</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:372</td><td>A*02:661</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:371</td><td>A*02:660</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:370N</td><td>A*02:659</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:369</td><td>A*02:658</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:364</td><td>A*02:656</td><td rowspan="2">A*24</td><td rowspan="2">A*02</td></tr>
<tr><td>99.97%</td><td>99.43%</td></tr>
<tr><td rowspan="2">Minor contig alleles</td><td>A*24:362</td><td>A*02:650</td><td rowspan="2">A*24</td><td rowspan="2">A*02</td></tr>
<tr><td>99.97%</td><td>99.43%</td></tr>
<tr><td rowspan="9">HLA_B</td><td rowspan="2">Major contig alleles</td><td>B*57:01:01:01</td><td>B*08:01:01:01</td><td rowspan="2">B*57</td><td rowspan="2">B*08</td></tr>
<tr><td>99.9%</td><td>100.0%</td></tr>
<tr><td rowspan="2">Minor contig alleles</td><td>B*57:03:01:02</td><td>B*08:01:01:02</td><td rowspan="2">B*57</td><td rowspan="2">B*08</td></tr>
<tr><td>99.85%</td><td>99.98%</td></tr>
<tr><td rowspan="2">Minor contig alleles</td><td>B*58:01:01:01</td><td>B*42:01:01</td><td rowspan="2">B*58</td><td rowspan="2">B*42</td></tr>
<tr><td>98.54%</td><td>99.58%</td></tr>
<tr><td rowspan="2">Minor contig alleles</td><td>B*58:01:01:03</td><td>B*48:01:01:01</td><td rowspan="2">B*58</td><td rowspan="2">B*48</td></tr>
<tr><td>98.53%</td><td>98.7%.</td></tr>
<tr><td>Minor contig</td><td>B*15:21:01:01</td><td>B*07:02:01:01</td><td>B*15</td><td>B*07</td></tr>
</table>

(continued)

| Gene | | Genetic allele | | Serological allele | |
|---|---|---|---|---|---|
| | alleles | 98.19% | 98.7%. | | |
| | Minor contig alleles | B*15:01:01:01 | B*07:05:01:01 | B*15 | B*07 |
| | | 97.97% | 98.68% | | |
| | Minor contig alleles | B*15:08:01 | B*07:03 | B*15 | B*07 |
| | | 97.95% | 98.75% | | |
| | Minor contig alleles | B*15:15 | B*07:05:01:03 | B*15 | B*07 |
| | | 97.92% | 98.62% | | |
| | Minor contig alleles | B*46:01:01 | B*39:10:01 | B*46 | B*39 |
| | | 97.9% | 98.38% | | |
| | Minor contig alleles | B*15:02:01 | B*39:01:03:01 | B*15 | B*39 |
| | | 98.06% | 98.38% | | |
| HLA_C | Major contig alleles | C*07:01:01:01 | C*06:02:01:01 | C*07 | C*06 |
| | Minor contig alleles | C*07:70 | C*06:58 | C*07 | C*06 |
| | | 99.98% | 99.98% | | |
| | Minor contig alleles | C*07:24 | C*06:07 | C*07 | C*06 |
| | | 99.98% | 99.98% | | |
| | Minor contig alleles | C*07:01:31 | C*06:02:20 | C*07 | C*06 |
| | | 99.98% | 99.98% | | |
| | Minor contig alleles | C*07:01:08 | C*06:02:01:12 | C*07 | C*06 |
| | | 99.98% | 99.98% | | |
| | Minor contig alleles | C*07:01:01:16 | C*06:02:01:02 | C*07 | C*06 |
| | | 99.98% | 99.95% | | |
| | Minor contig alleles | C*07:250 | C*06:06 | C*07 | C*06 |
| | | 99.98% | 99.93% | | |
| | Minor contig alleles | C*07:40 | C*12:03:01:01 | C*07 | C*12 |
| | | 99.95% | 99.74% | | |
| | Minor contig alleles | C*07:22 | C*12:20 | C*07 | C*12 |
| | | 99.95% | 99.7% | | |
| | Minor contig alleles | C*07:212 | C*12:18:01 | C*07 | C*12 |
| | | 99.95% | 99.63% | | |

(continued)

| Gene | | Genetic allele | | Serological allele | |
|---|---|---|---|---|---|
| HLA_D RB1 | Major contig alleles | DRB1*15:03:01: 01 | DRB1*15:03:01: 01 | DRB1*15 | DRB1*15 |
| | | 94.1% | 94.38% | | |
| | Minor contig alleles | DRB1*15:03:01: 02 | DRB1*15:03:01 : 02 | DRB1*15 | DRB1*1 5 |
| | | 94.07% | 94.35% | | |
| | Minor contig alleles | DRB1*15:01:01: 02 | DRB1*15:01:01: 02 | DRB1*1 5 | DRB1*1 5 |
| | | 94.05% | 94.35% | | |
| | Minor contig alleles | DRB1*15:01:01: 05 | DRB1*15:01:01: 05 | DRB1*1 5 | DRB1*1 5 |
| | | 94.08% | 94.38% | | |
| | Minor contig alleles | DRB1*15:04 | DRB1*15:04 | DRB1*1 5 | DRB1*1 5 |
| | | 94.03% | 94.33% | | |
| | | DRB1*15:02:01: 03 | DRB1*15:02:01: 03 | DRB1*15 | DRB1*1 |
| | Minor contig alleles | 93.93% | 94.21% | | 5 |
| | Minor contig alleles | DRB1*16:02:01: 03 | DRB1*16:02:01: 03 | DRB1*1 6 | DRB1*6 |
| | | 93.98% | 94.29% | | |
| | Minor contig alleles | DRB1*15:02:01: 02 | DRB1*15:02:01: 02 | DRB1*1 5 | DRB1*1 5 |
| | | 93.92% | 94.19% | | |
| | Minor contig alleles | DRB1*16:02:01: 02 | DRB1*16:02:01: 02 | DRB1*1 6 | DRB1*1 6 |
| | | 93.97% | 94.27% | | |
| | Minor contig alleles | DRB1*16:01:01 | DRB1*16:01:01 | DRB1*1 6 | DRB1*1 6 |
| | | 93.97% | 94.27% | | |
| | Minor contig alleles | DPA1*02:07:01: 01 | DPA1*02:07:01: 01 | DPA1*0 2 | DPA1*0 2 |
| | | 99.39% | 99.39% | | |
| | Major contig alleles | DPA1*02:01:01: 01 | DPA1*02:01:01: 01 | DPA1*0 2 | DPA1*0 2 |
| | | 99.97% | 99.97% | | |
| | Minor contig alleles | DPA1*02:01:01: 03 | DPA1*02:01:01: 03 | DPA1*0 2 | DPA1*0 2 |
| | | 99.95% | 99.95% | | |
| | Minor contig alleles | DPA1*02:01:01: 02 | DPA1*02:01:01: 02 | DPA1*0 2 | DPA1*0 2 |
| | | 99.95% | 99.95% | | |

(continued)

| Gene | | Genetic allele | | Serological allele | |
|---|---|---|---|---|---|
| HLA_D PA1 | Minor contig alleles | DPA1*02:08 | DPA1*02:08 | DPA1*0 2 | DPA1*0 2 |
| | | 99.94% | 99.94% | | |
| | Minor contig alleles | DPA1*02:02:02:01 | DPA1*02:02:02:01 | DPA1*0 2 | DPA1*0 2 |
| | | 99.28% | 99.28% | | |
| | Minor contig alleles | DPA1*02:02:02:03 | DPA1*02:02:02:03 | DPA1*0 2 | DPA1*0 2 |
| | | 99.27% | 99.27% | | |
| | Minor contig alleles | DPA1*02:02:02:02 | DPA1*02:02:02:02 | DPA1*0 2 | DPA1*0 2 |
| | | 99.27% | 99.27% | | |
| | Minor contig alleles | DPA1*01:03:01:02 | DPA1*01:03:01:02 | DPA1*0 1 | DPA1*0 1 |
| | | 96.34% | 96.34% | | |
| | Minor contig alleles | DPA1*01:03:01 :03 | DPA1*01:03:01:03 | DPA1*0 1 | DPA1*0 1 |
| | | 96.33% | 96.33% | | |
| HLA_D PB1 | Major contig alleles | DPB1*01:01:01:01 | DPB1*01:01:01:01 | DPB1*0 1 | DPB1*0 1 |
| | | 99.98% | 99.98% | | |
| | Minor contig alleles | DPB1*667:01 | DPB1*667:01 | DPB1*6 67 | DPB1*6 67 |
| | | 99.97% | 99.97% | | |
| | Minor contig alleles | DPB1*127:01 | DPB1*127:01 | DPB1*1 27 | DPB1*1 27 |
| | | 99.97% | 99.97% | | |
| | Minor contig alleles | DPB1*01:01:01:03 | DPB1*01:01:01:03 | DPB1*0 1 | DPB1*0 1 |
| | | 99.97% | 99.97% | | |
| | | DPB1*01:01:01:02 | DPB1*01:01:01:02 | DPB1*0 | DPB1*0 |

(continued)

| Gene | | Genetic allele | | Serological allele | |
|---|---|---|---|---|---|
| | Minor contig alleles | 99.94% | 99.94% | 1 | 1 |
| | Minor contig alleles | DPB1*90:01:01 | DPB1*90:01:01 | DPB1*9 0 | DPB1*9 0 |
| | | 99.93% | 99.93% | | |
| | Minor contig alleles | DPB1*01:01:01:04 | DPB1*01:01:01:04 | DPB1*0 1 | DPB1*0 1 |
| | | 99.9% | 99.9% | | |
| | Minor contig alleles | DPB1*26:01:02 | DPB1*26:01:02 | DPB1*2 6 | DPB1*2 6 |
| | | 99.88% | 99.88% | | |
| | Minor contig alleles | DPB1*01:01:02:02 | DPB1*01:01:02:02 | DPB1*0 1 | DPB1*0 1 |
| | | 99.86% | 99.86% | | |
| | Minor contig alleles | DPB1*01:01:02:01 | DPB1*01:01:02:01 | DPB1*0 1 | DPB1*0 1 |
| | | 99.84% | 99.84% | | |
| HLA D QA1 | Major contig alleles | DQA1*05:01:01:02 | DQA1*05:01:01:02 | DQA1* 05 | DQA1* 05 |
| | | 98.3% | 95.21% | | |
| | Minor contig alleles | DQA1*05:03:01:02 | DQA1*05:03:01:02 | DQA1* 05 | DQA1* 05 |
| | | 98.29% | 95.2% | | |
| | Minor contig alleles | DQA1*05:07 | DQA1*05:07 | DQA1* 05 | DQA1* 05 |
| | | 98.27% | 95.18% | | |
| | Minor contig alleles | DQA1*05:06:01:01 | DQA1*05:06:01:01 | DQA1* 05 | DQA1* 05 |
| | | 98.27% | 95.18% | | |
| | Minor contig alleles | DQA1*05:01:01:01 | DQA1*05:01:01:01 | DQA1* 05 | DQA1* 05 |
| | | 98.27% | 95.18% | | |
| | Minor contig alleles | DQA1*05:06:01:02 | DQA1*05:06:01:02 | DQA1* 05 | DQA1* 05 |
| | | 98.26% | 98.17% | | |
| | Minor contig alleles | DQA1*05:05:01:03 | DQA1*05:05:01:03 | DQA1* 05 | DQA1* 05 |
| | | 97.06% | 94.2% | | |
| | Minor contig alleles | DQA1*05:05:01:09 | DQA1*05:05:01:09 | DQA1* 05 | DQA1* 05 |
| | | 97.0% | 94.14% | | |
| | Minor contig alleles | DQA1*05:11 | DQA1*05:11 | DQA1* 05 | DQA1* 05 |
| | | 96.98% | 94.12% | | |

(continued)

<table>
<tr><td>Gene</td><td></td><td colspan="2">Genetic allele</td><td colspan="2">Serological allele</td></tr>
<tr><td rowspan="2"></td><td rowspan="2">Minor contig alleles</td><td>DQA1*05:05:01:01</td><td>DQA1*05:05:01:01</td><td rowspan="2">DQA1* 05</td><td rowspan="2">DQA1* 05</td></tr>
<tr><td>96.94%</td><td>94.08%</td></tr>
<tr><td colspan="6">4. Reference transcripts used in the analysis of the results<br>HLA-A (NM_002116.7); HLA-B (NM_005514.6); HLA-C (NM_002117.5); HLA-DRB1 (NM_002124.3); HLA-DQB1 (NM_002123.4); HLA-DPB1 (NM_002121.5); HLA-DQA1 (NM_002122.3); HLA-DPA1 (NM_033554); HLA-DRB3 (NM_022555); HLA-DRB4 (NM_021983); HLA-DRB5 (NM_002125)</td></tr>
<tr><td colspan="6">5. Notes<br>Release IMGT-HLA</td></tr>
</table>

**CITATION LIST**

Bauer, D. F. (1972). Constructing confidence sets using rank statistics. Journal of the American Statistical Association, 67, 687-690

Felsenstein, J. (1981). Evolutionary trees from DNA sequences: a maximum likelihood approach. Journal of Molecular Evolution, 17(6):368-376

Gascuel, O. (1997). Concerning the NJ algorithm and its unweighted version, UNJ. in Birkin et. al. Mathematical Hierarchies and Biology, 149-170

Gu, X. et al. (1999). Locus Specificity of Polymorphic Alleles and Evolution by a Birth-and-Death Process in Mammalian MHC Genes. Mol Biol Evol, 16(2):147-56

Hosomichi, K. et al. (2013). Phase-defined complete sequencing of the HLA genes by next-generation sequencing. BMC Genomics, May 28;14:355

Katoh, K. et al. (2013). MAFFT Multiple Sequence Alignment Software Version 7: Improvements in Performance and Usability. Mol Biol Evol, 30(4):772-80

Li, H. et al. (2009). Fast and accurate short read alignment with Burrows-Wheeler transform. Bioinformatics, 25(14), 1754-1760

McKenna, A. et al. (2010). The Genome Analysis Toolkit: a MapReduce framework for analyzing next-generation DNA sequencing data. Genome Res., 20(9):1297-303

McKenzie, L. M. et al. (1999). Taxonomic hierarchy of HLA class I allele sequences. Genes Immun. Nov;1(2):120-9.

Patterson, M. et al. (2015). WhatsHap: Weighted Haplotype Assembly for Future-Generation Sequencing Reads. J. Comput. Biol., 22(6):498-509

Pearson, W. R. (2016). Finding Protein and Nucleotide Similarities with FASTA. Curr Protoc Bioinformatics, 53:3.9.1-25

Rousseeuw, P. J. (1987). Silhouettes: a Graphical Aid to the Interpretation and Validation of Cluster Analysis. Journal of Computational and Applied Mathematics, 20, 53-65

Saitou, N. et al. (1987). The neighbor-joining method: a new method for reconstructing phylogenetic trees. Molecular Biology and Evolution, 4, 406-425

Schliep K. P. (2011). phangorn: phylogenetic analysis in R. Bioinformatics, 27(4), 592-593

Schliep, K. P. et al. (2017). Intertwining phylogenetic trees and networks. Methods in Ecology and Evolution, 8:1212-1220

Shiina, T. et al. (2012). Super high resolution for single molecule-sequence-based typing of classical HLA loci at the 8-digit level using next generation sequencers. Tissue Antigens, 80(4):305-16

Sneath, P.H.A. et al. (1973). Numerical Taxonomy: The Principles and Practice of Numerical Classification, p. 278 ff; Freeman, San Francisco.

Studier, J. A. et al. (1988). A Note on the Neighbor-Joining Algorithm of Saitou and Nei. Molecular Biology and Evolution, 6, 729-731

## Claims

**1.** A method for genotypically identifying both alleles of at least one locus of a subject's HLA gene comprising:

(a) amplifying genomic DNA samples of said subject by long-range PCR using at least one pair of sense and antisense primers for each sample wherein said pair of sense and antisense primers is identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, wherein said pair of primers amplifies in a single amplicon at least one complete HLA gene together with 5'-UTR and 3'-UTR regions of said HLA gene, an upstream region and a downstream region of said HLA gene, of both alleles, of at least one locus of an HLA gene, thus forming amplicons, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1,
(b) combining the amplicons from each of the samples,

(c) fragmenting the amplicons, thus obtaining fragments and labelling said fragments, thus forming libraries of labelled fragments,
(d) combining said libraries of labelled fragments,
(e) sequencing said libraries of labelled fragments by Next Generation sequencing, thereby generating reads of partially overlapping sequences,
(f) aligning said sequence reads against a reference genome,
(g) identifying variants based on variant calling,
(h) determining haplotype blocks based on the alignment of the sequences reads against a reference genome and adding flanking regions of said haplotype blocks to said haplotype blocks,
(i) locally aligning the sequence reads of the haplotype blocks obtained in the previous step against all the alleles of a reference database of genomic sequences encoding HLA genes for each HLA gene, thus obtaining first candidate alleles,
(j) pre-calculating the genetic distances and determining the phylogenetic grouping of the HLA families at different levels in the reference database of genomic sequences that encode HLA genes, thus obtaining a distance matrix containing the distance values of each allele with itself and with the others,
(k) carrying out a primary selection of said first candidate alleles based on the overall homology contrast result of the sequence reads to the haplotypes, thus obtaining second candidate alleles,
(l) carrying out a study of evolutionary phylogeny of said second candidate alleles, thus obtaining a phylogenetic tree, accessing the distance matrix obtained in step (j) and selecting HLA alleles from the reference database of genomic sequences encoding the phylogenetically closest HLA genes, thus obtaining contrasted HLA alleles and
(m) carrying out a contrast test with contrasted HLA alleles, calculating the distances between the sequence reads and the HLA alleles contrasted in each level and assigning each reading of sequences to a known HLA family at different levels, wherein a sequence reading is assigned to a known HLA family at different levels when the statistical significance obtained in said contrast test is above a defined cut-off value and wherein a sequence reading is assigned as a *de novo* sequence that codes for a new locus of the HLA gene when the statistical significance obtained in said contrast test is below a defined cut-off value.

**2.** The method according to claim 1 wherein said statistical significance is calculated based on the statistical value p.

**3.** The method according to claim 1 or 2 wherein said defined cut-off value is calculated based on the statistical value p and has a value of $1^{-6}$.

**4.** The method according to any of the claims 1 to 3 wherein the length of the fragments generated in step (c) is between 1200 and 1500 bp.

**5.** The method according to any of the claims 1 to 4, wherein the libraries are sequenced in step (e) by Next Generation sequencing with a reading length of between 200 and 300 bp.

**6.** A pair of primers identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, for use in the genotypic identification of both alleles of at least one locus of a subject's HLA gene, wherein said pair of primers amplifies in a single amplicon a complete HLA gene together with 5'-UTR and 3'-UTR regions of said HLA gene, an upstream region and a downstream region of said HLA gene, of both alleles of at least one locus of an HLA gene, wherein said HLA gene is selected, respectively, from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1.

**7.** A kit for genotypically identifying both alleles of at least one locus of a subject's HLA gene comprising:

(a) at least one pair of sense and antisense primers, identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22, wherein said pair of primers amplifies in a single amplicon a complete HLA gene together with 5'-UTR and 3'-UTR regions of said HLA gene, an upstream region and a downstream region of said HLA gene, of both alleles of at least one locus of the HLA gene, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1,

(b) a DNA polymerase and
(c) instructions for use.

**8.** The kit according to claim 7, additionally comprising reagents for amplification and sequencing.

**9.** The kit according to claim 7 or 8 for use in genotypic identification of both alleles of at least one locus of an HLA gene wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1.

**10.** A computer program product comprising a computer readable medium, wherein there are coded instructions for controlling a computer program system comprising the following means for carrying out a genotypic identification of both alleles of at least one locus of a subject's HLA gene, wherein said genotypic identification comprises amplifying genomic DNA samples of said subject by long-range PCR using at least one pair of sense and antisense primers, for each sample, wherein said pair of sense and antisense primers is identified by the sequences selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20 and SEQ ID NO: 21-22; wherein said pair of primers amplifies in a single amplicon at least one complete HLA gene together with 5'-UTR and 3'-UTR regions of said HLA gene, an upstream region and a downstream region of said HLA gene, of both alleles, of at least one locus of an HLA gene, thus forming amplicons, wherein said HLA gene is selected from the group consisting of HLA-A, HLA-B, HLA-C, HLA-DRB1, HLA-DRB3, HLA-DRB4, HLA-DRB5, HLA-DPB1, HLA-DQB1, HLA-DPA1 and HLA-DQA1, combining the amplicons for each of the samples, fragmenting the amplicons, thus obtaining fragments and labelling said fragments, thus forming libraries of labelled fragments, combining said libraries of labelled fragments, sequencing said libraries of labelled fragments by Next Generation sequencing, thus generating sequence reads that partially overlap and wherein said system comprises the following means:

(a) means for carrying out an alignment of said sequence reads against a reference genome,
(c) means for identifying variants based on a variant calling,
(d) means for determining haplotype blocks based on the alignment of sequence reads against a reference genome and means for adding flanking regions of said haplotype blocks to said haplotype blocks,
(e) means for locally aligning the sequence reads of the haplotype blocks obtained in the previous step against all the alleles of a reference database of genomic sequences encoding HLA genes for each HLA gene, thus obtaining first candidate alleles,
(f) means for pre-calculating the genetic distances and determining the phylogenetic grouping of the HLA families at different levels in the reference database of genomic sequences that encode HLA genes, thus obtaining a distance matrix containing the distance values of each allele with itself and with the others,
(g) means for carrying out a primary selection of said first candidate alleles based on the overall homology contrast result of the sequence reads to the haplotypes, thus obtaining second candidate alleles,
(h) means for carrying out an evolutionary phylogeny study of said second candidate alleles, thus obtaining a phylogenetic tree, means for accessing the distance matrix obtained in step (f) and means for selecting HLA alleles from the reference database of genomic sequences that encode phylogenetically closest HLA genes, thereby obtaining contrasted HLA alleles,
(i) means for carrying out a contrast test with contrasted HLA alleles and
(j) means for calculating the distances between the sequence reads and the contrasted HLA alleles at each level and means for assigning each sequence reading to a known HLA family at different levels, wherein a sequence reading is assigned to a known HLA family at different levels when the statistical significance obtained in said contrast test is above a defined cut-off value and wherein a sequence reading is assigned as a *de novo* sequence that codes for a new locus of the HLA gene when the statistical significance obtained in said contrast test is below a defined cut-off value.

**11.** The computer program product according to claim 10 wherein said statistical significance is calculated based on the statistical value p.

**12.** The computer program product of claim 10 or 11 wherein said defined cut-off value is calculated based on the statistical value p and has a value of $1^{-6}$.

FIGURE 1

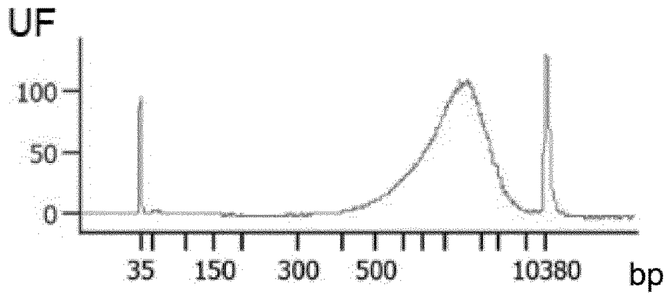

FIGURE 2

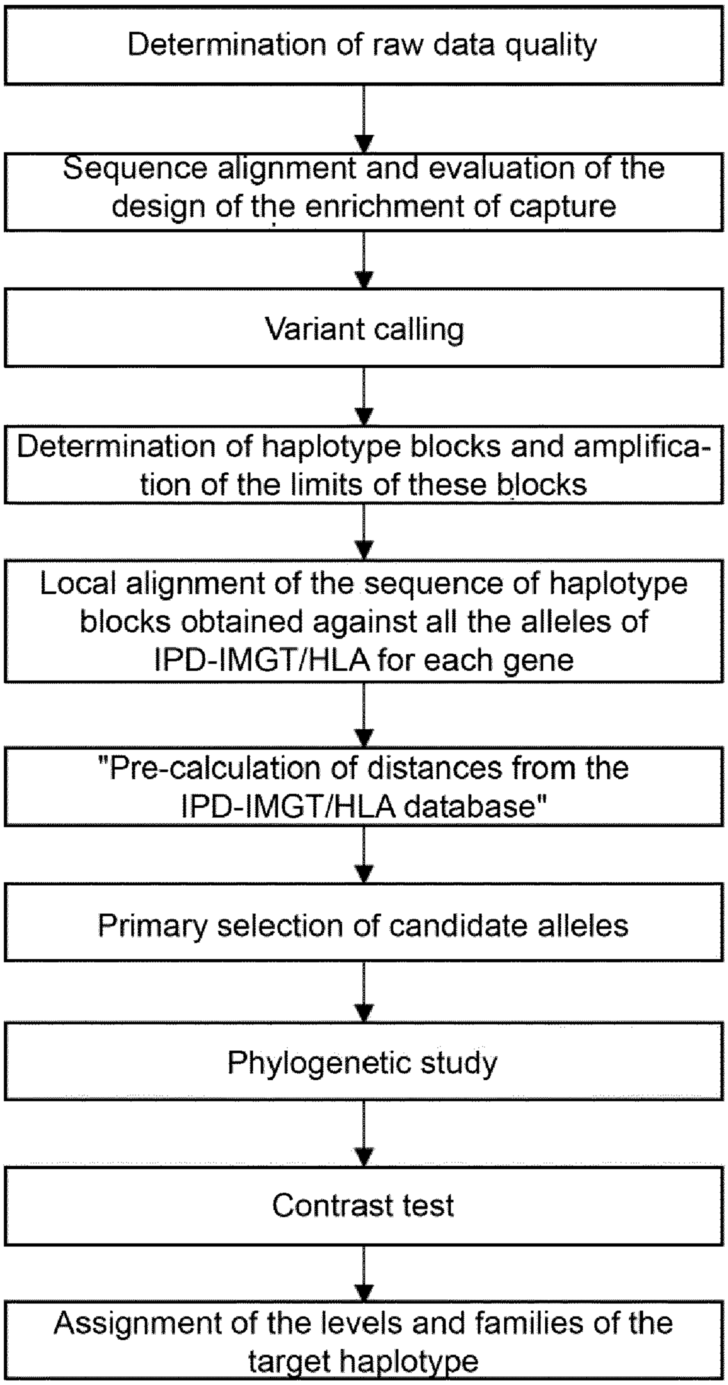
Determination of raw data quality
Sequence alignment and evaluation of the design of the enrichment of capture
Variant calling
Determination of haplotype blocks and amplification of the limits of these blocks
Local alignment of the sequence of haplotype blocks obtained against all the alleles of IPD-IMGT/HLA for each gene
"Pre-calculation of distances from the IPD-IMGT/HLA database"
Primary selection of candidate alleles
Phylogenetic study
Contrast test
Assignment of the levels and families of the target haplotype

FIGURE 3

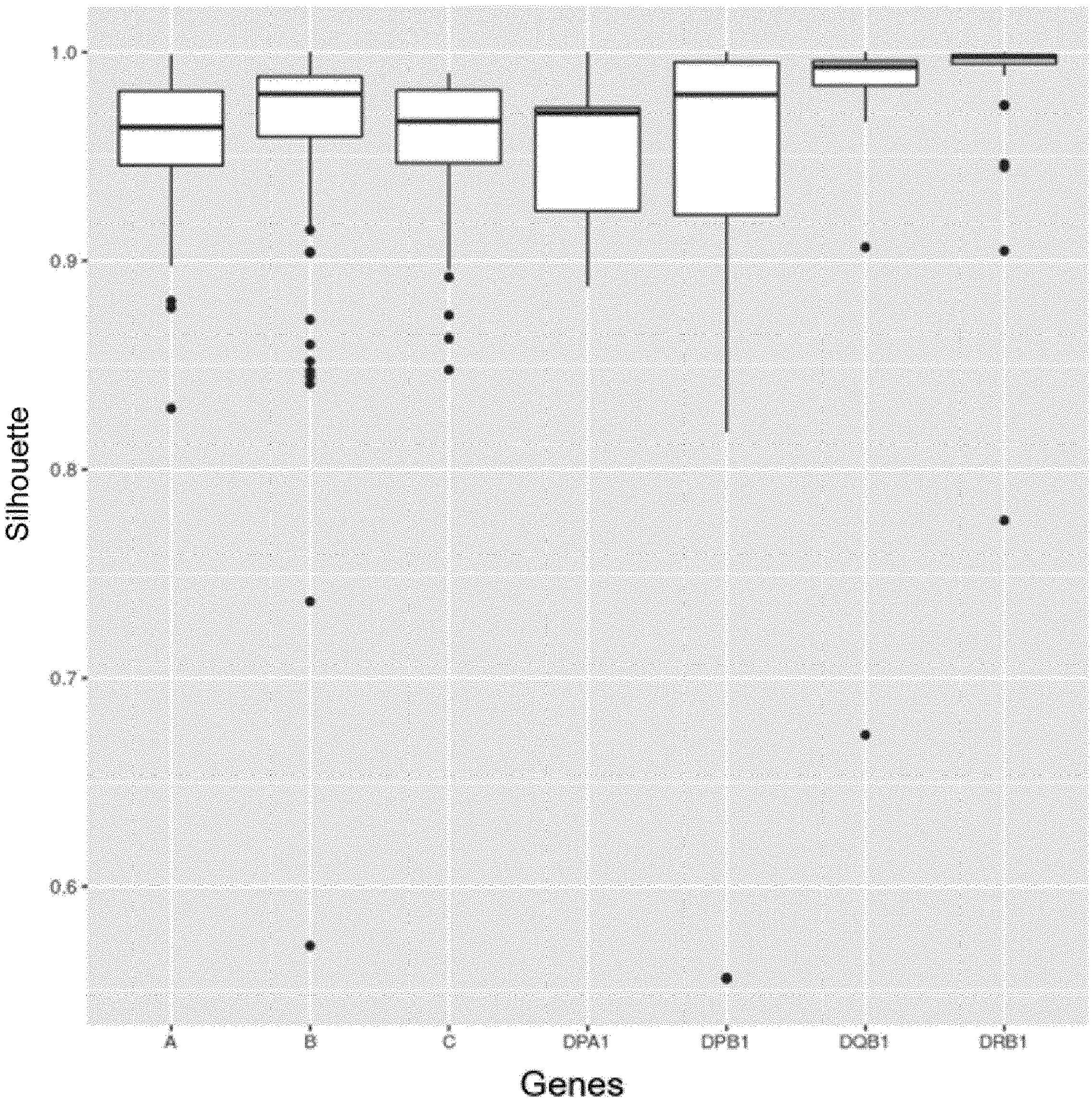

FIGURE 4

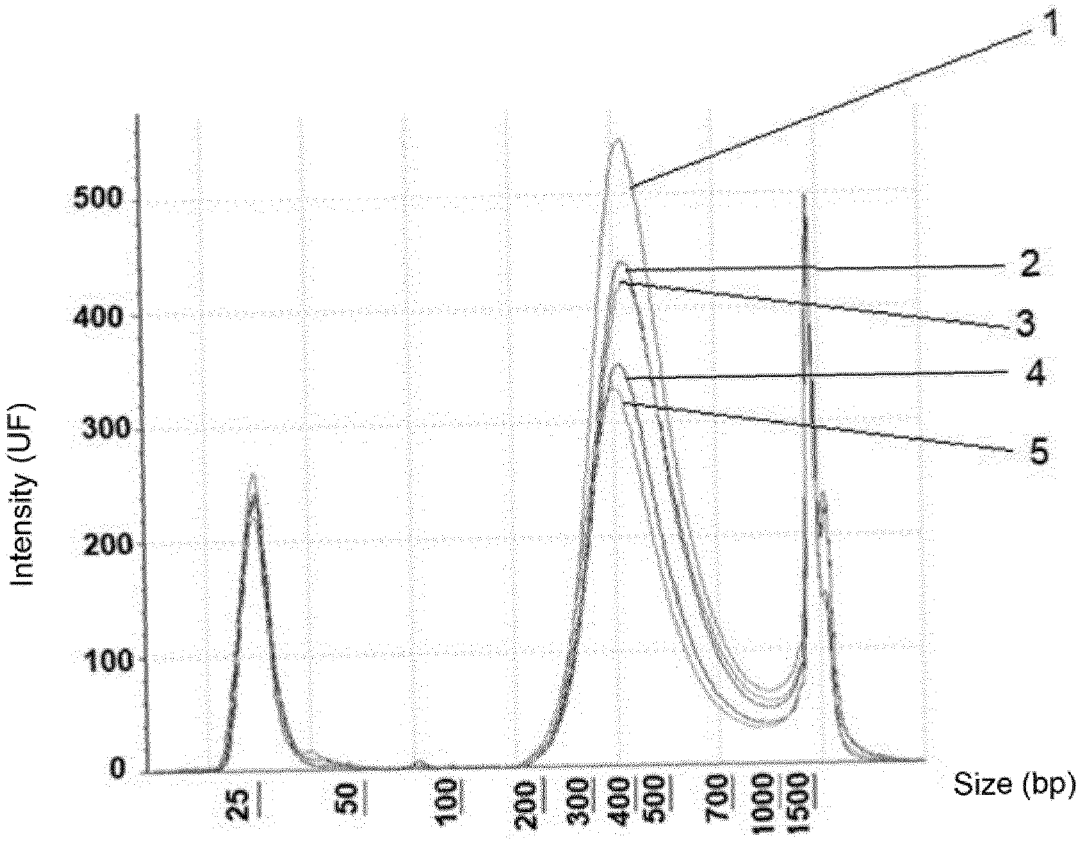
1
2
3
4
5
500
400
300
200
100
0
Intensity (UF)
25
50
100
200
300
400
500
700
1000
1500
Size (bp)

FIGURE 5

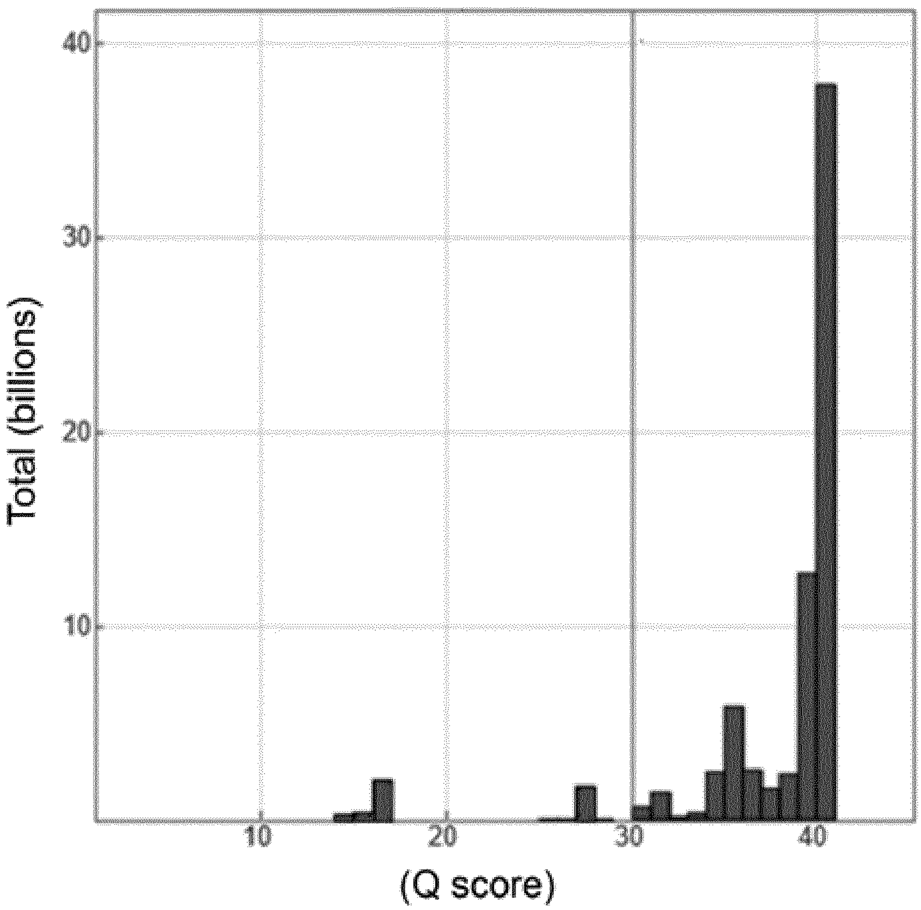

FIGURE 6

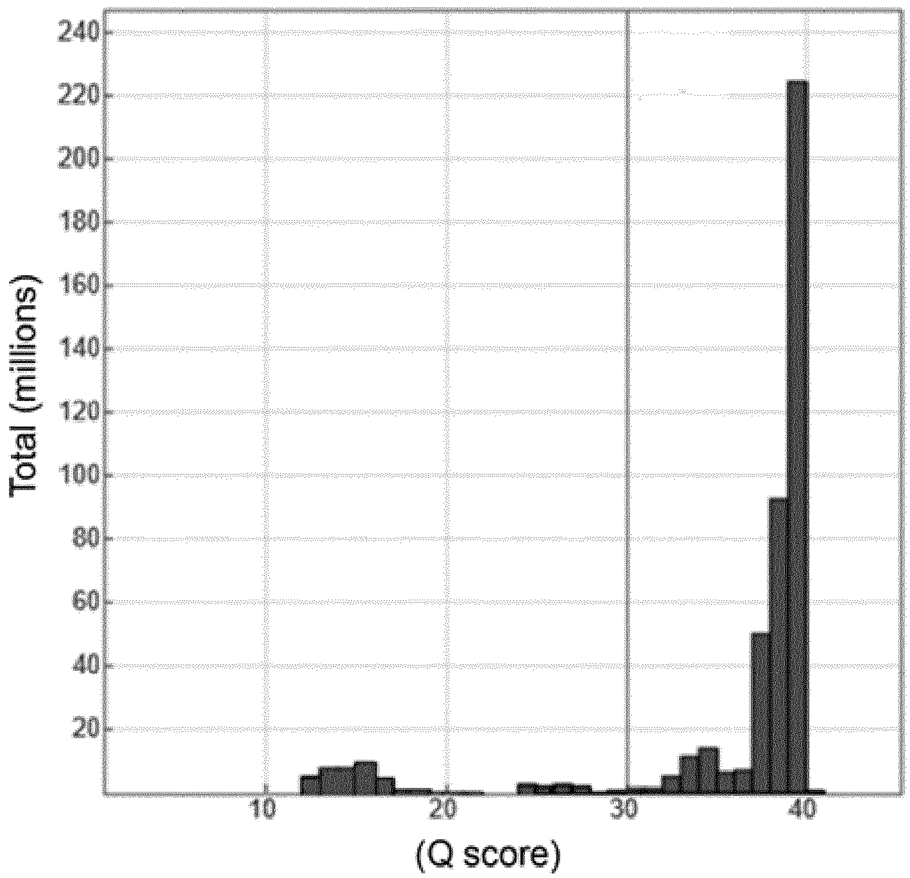


FIGURE 7

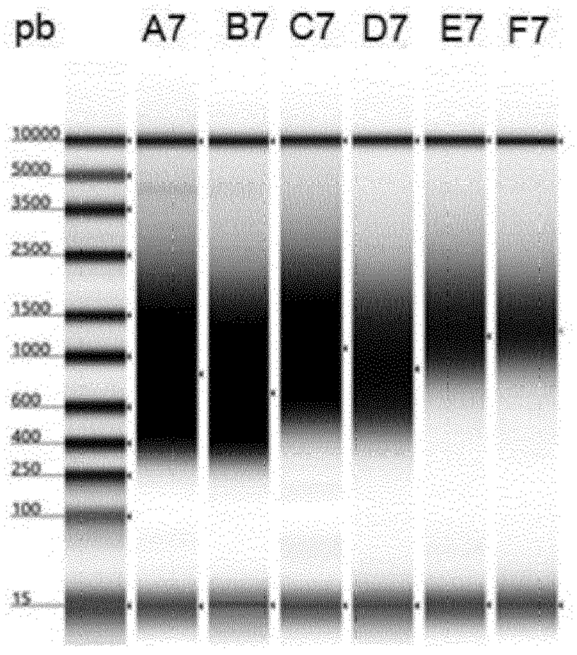

FIGURE 8

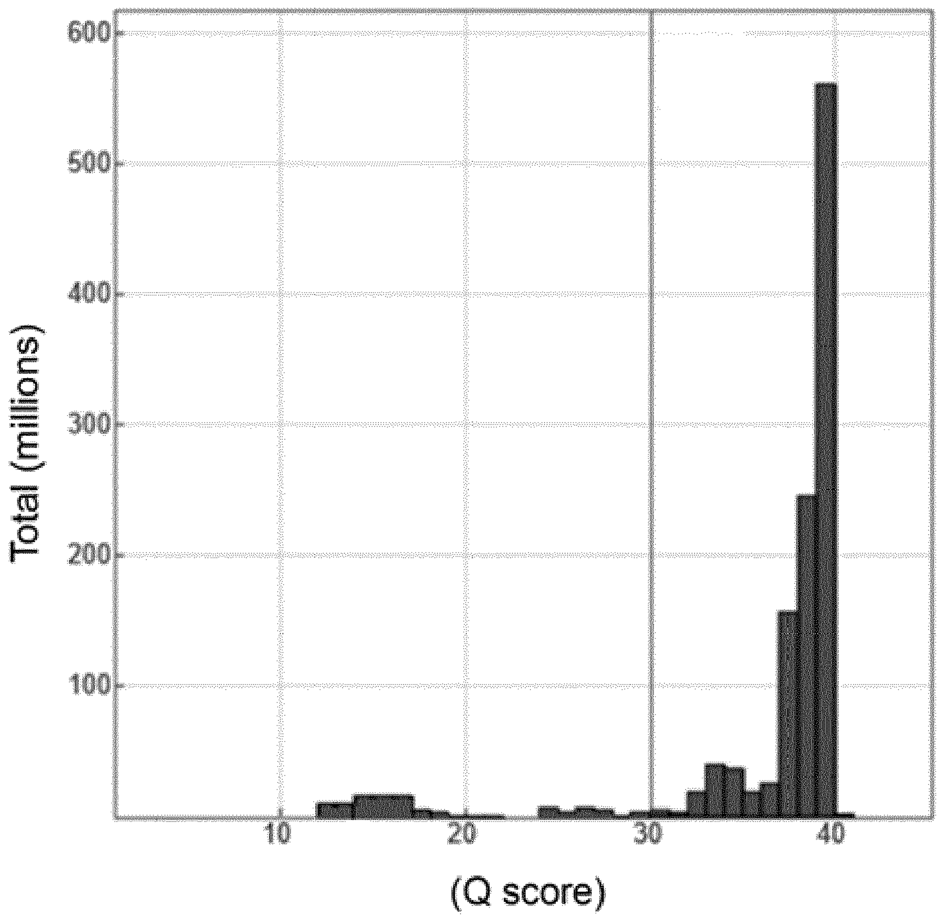

FIGURE 9

A

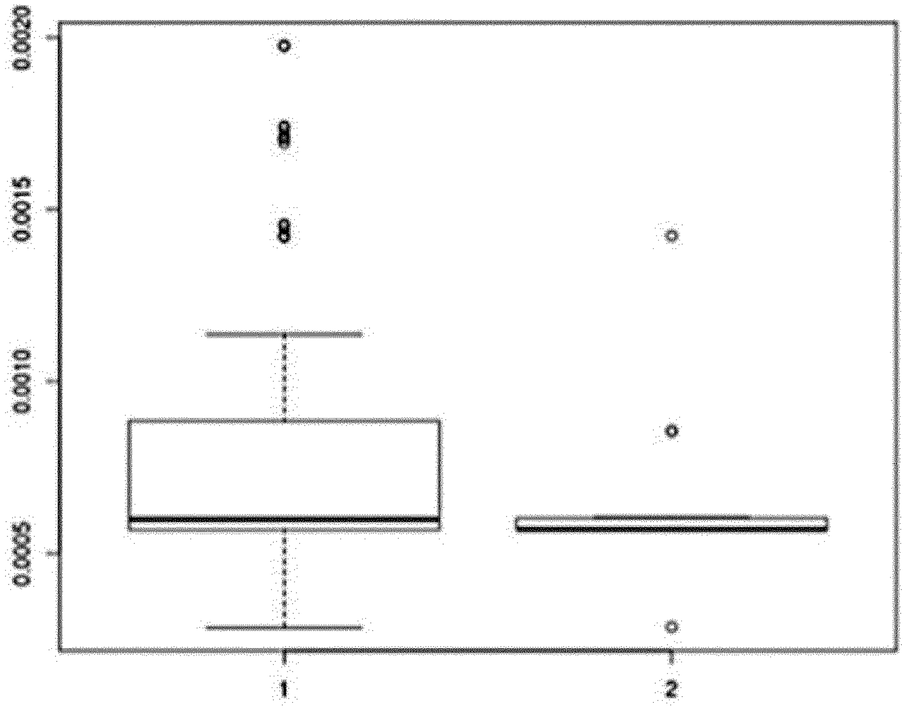
0.0020
0.0015
0.0010
0.0005
1
2

B

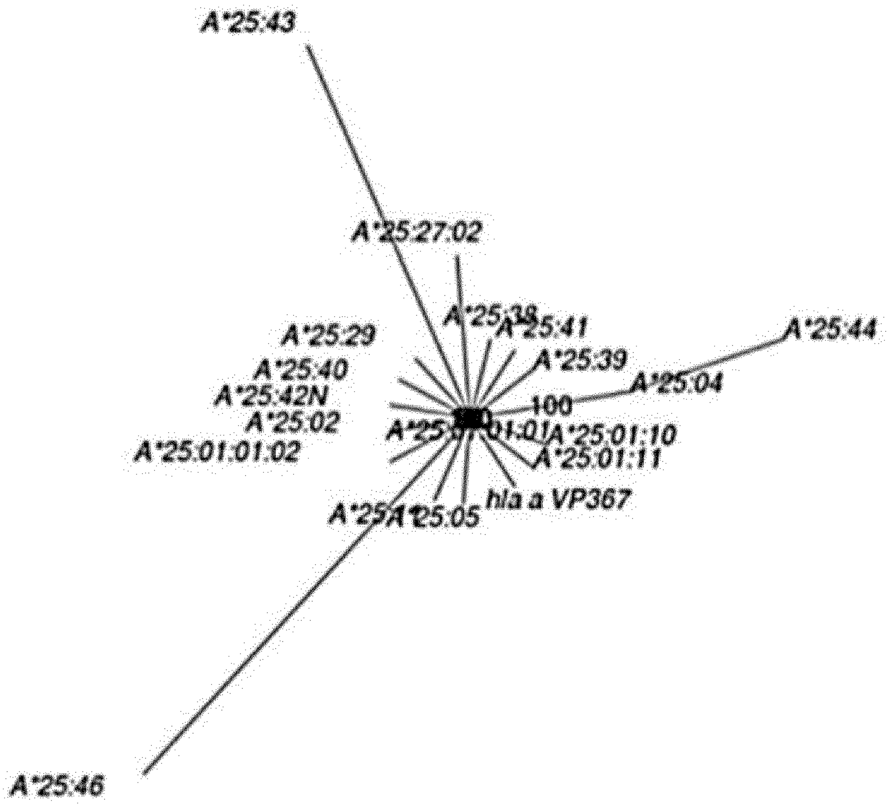
A*25:43
A*25:27:02
A*25:38
A*25:41
A*25:44
A*25:29
A*25:39
A*25:40
A*25:04
A*25:42N
100
A*25:02
A*25:01:10
A*25:01:01:02
A*25:01:11
hla a VP367
A*25:05
A*25:46

FIGURE 10

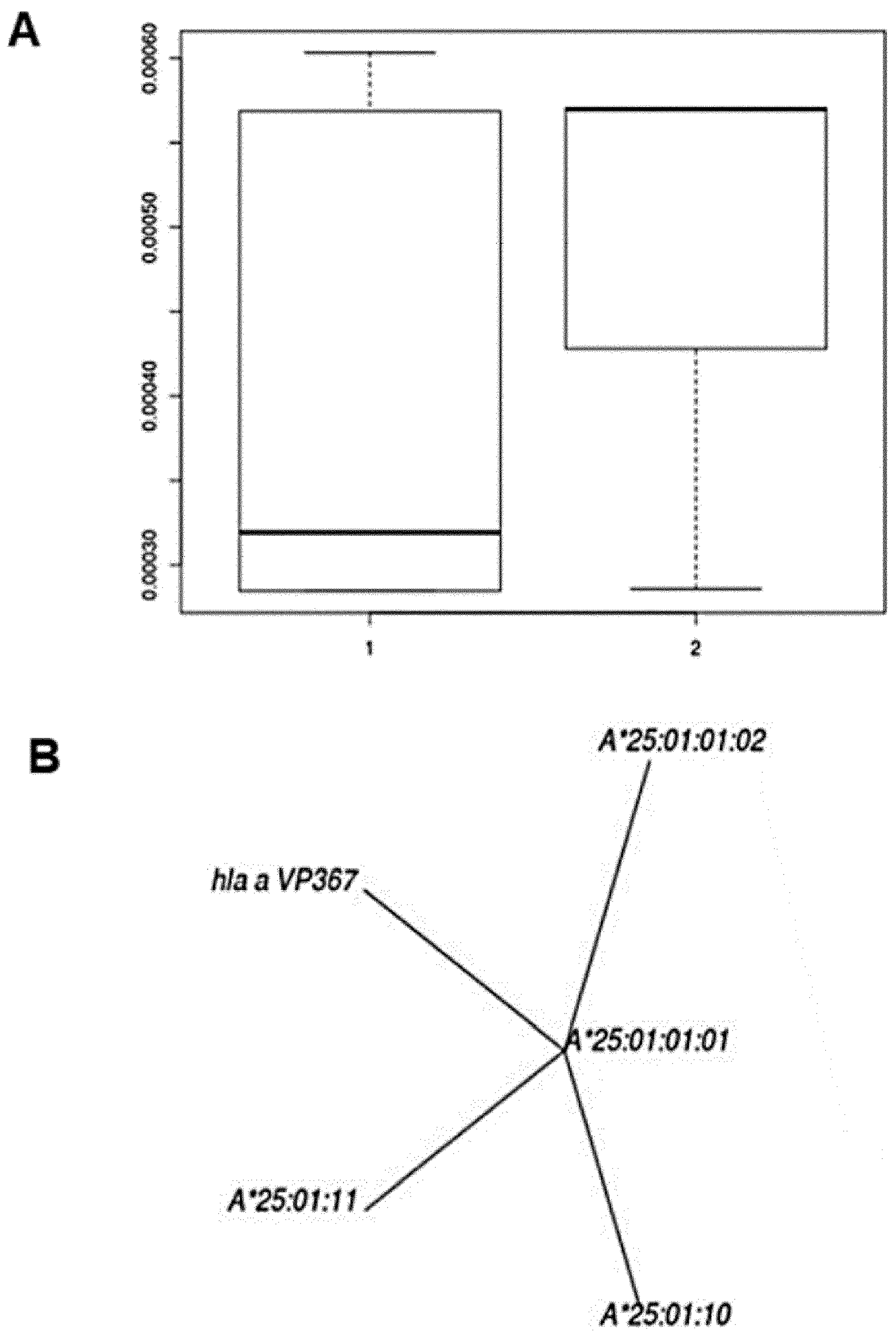
A
0.00060
0.00050
0.00040
0.00030
1
2
B
A*25:01:01:02
hla a VP367
A*25:01:01:01
A*25:01:11
A*25:01:10

# FIGURE 11

A

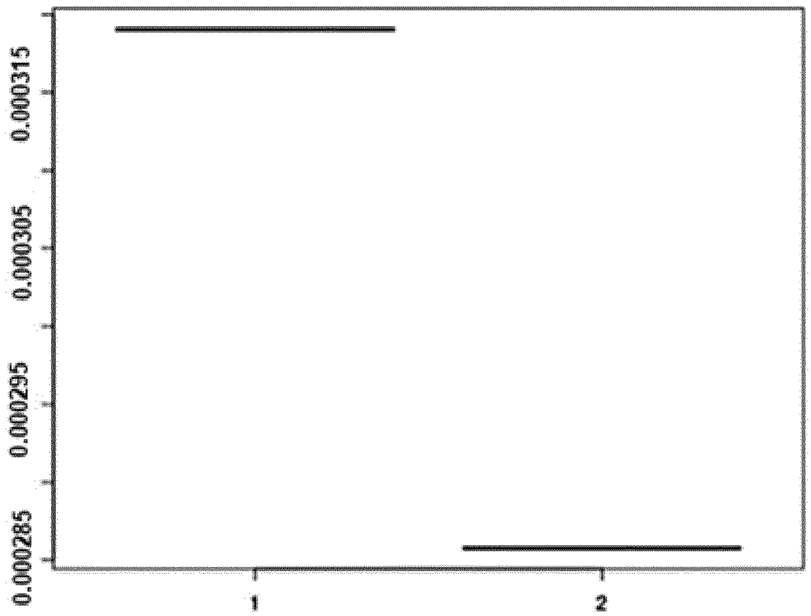
0.000315
0.000305
0.000295
0.000285
1
2

B

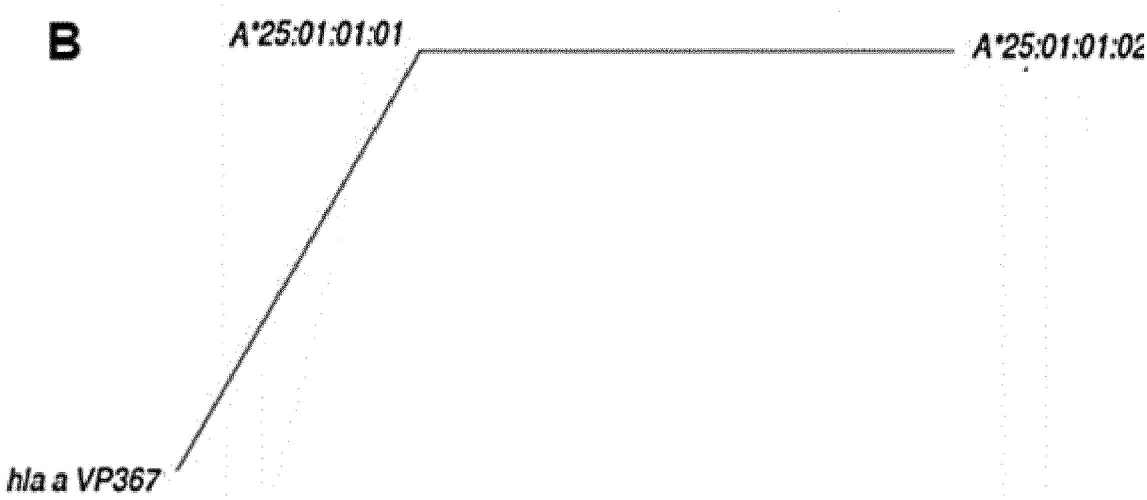
A*25:01:01:01
A*25:01:01:02
hla a VP367

FIGURE 12

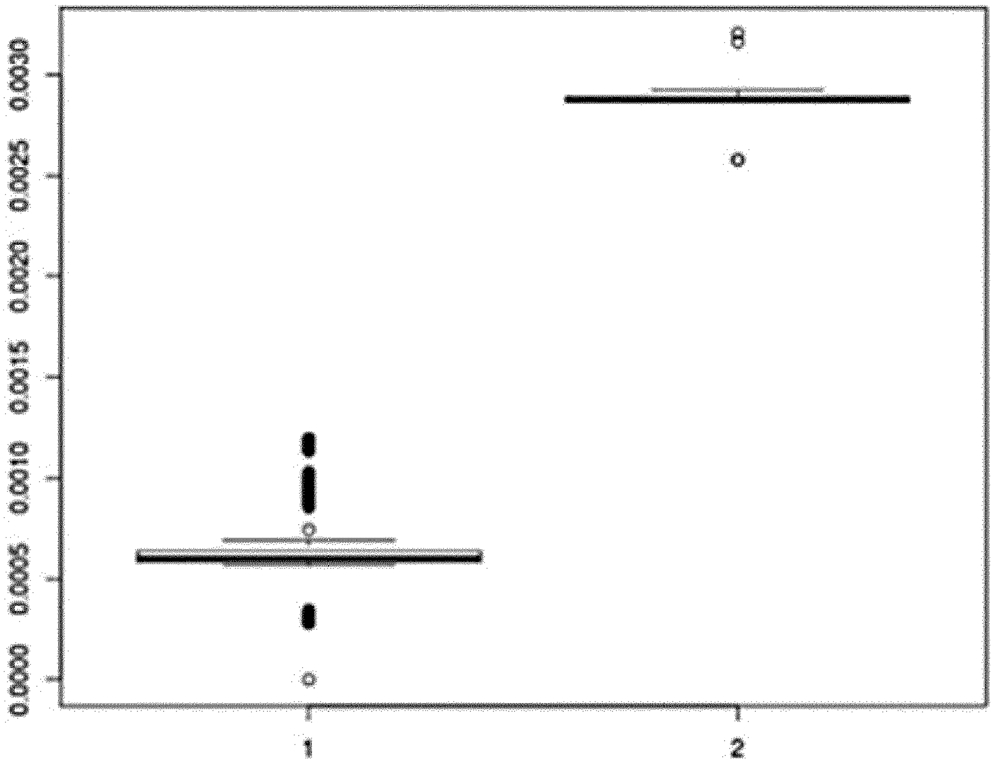

FIGURE 13

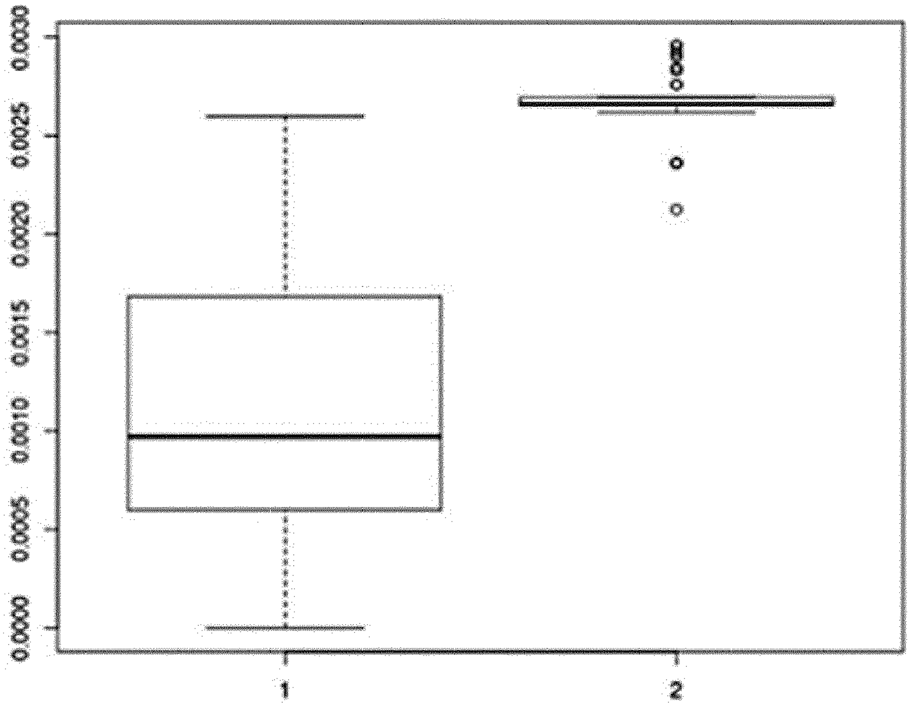

FIGURE 14

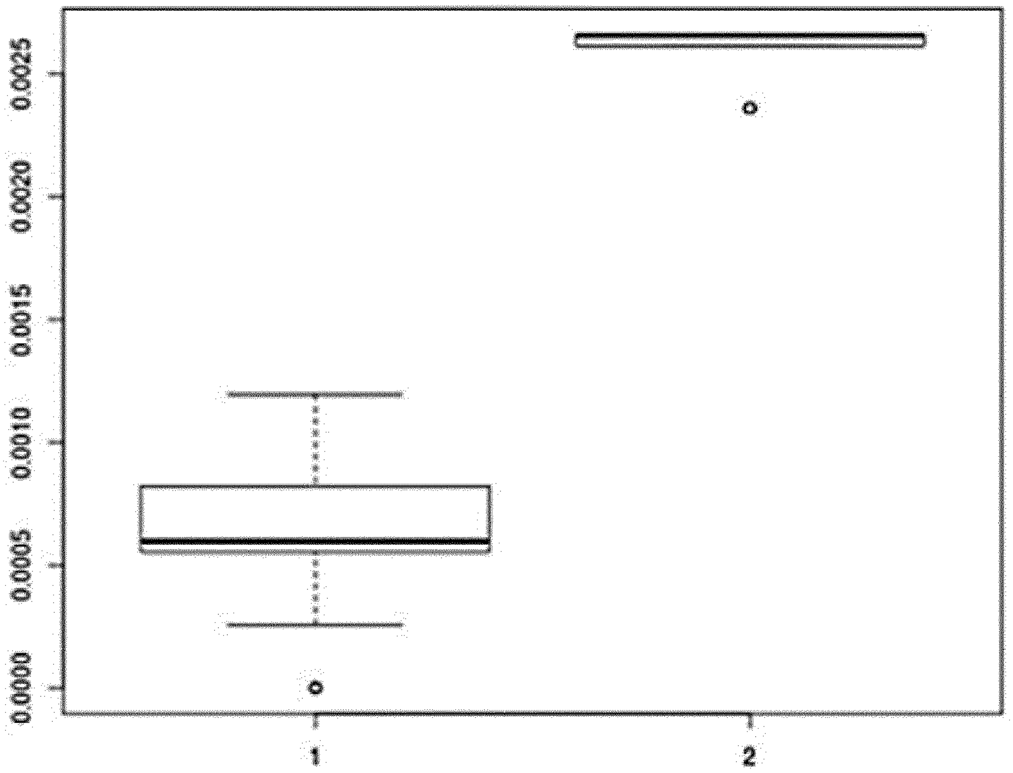

Europäisches Patentamt
European Patent Office
Office européen des brevets

# EUROPEAN SEARCH REPORT

Application Number
EP 18 38 2671

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 2 735 617 A1 (GENODIVE PHARMA INC [JP]) 28 May 2014 (2014-05-28) * SEQ ID NO:42 corresponds to SEQ ID NO:15 SEQ ID NO:45 corresponds to SEQ ID NO:16 SEQ ID NO:3 corresponds to SEQ ID NO:2; claim 33 * ----- | 6-9 | INV. C12Q1/6858 C12Q1/6881 |
| A | WO 2018/147438 A1 (UNIV KYOTO [JP]) 16 August 2018 (2018-08-16) * Human HLA-DRB1 gene reverse multiplex PCR primer (HLA-DRB1_R2), SEQ:24 =SEQ ID NO:8 of the present application * ----- | 1 | |
| A | WO 2018/058114 A1 (SIRONA GENOMICS INC [US]) 29 March 2018 (2018-03-29) * claim 1 * ----- | 1 | |
| A | US 2008/172209 A1 (HECKERMAN DAVID E [US] ET AL) 17 July 2008 (2008-07-17) * claims 1,15 * ----- | 1 | TECHNICAL FIELDS SEARCHED (IPC) |
| A | WO 2010/019550 A2 (SHIRAZ PHARMACEUTICALS INC [US]; ROSES ALLEN D [US]) 18 February 2010 (2010-02-18) * page 2, lines 5-22 * ----- | 1 | C12Q |

The present search report has been drawn up for all claims

1

| Place of search | Date of completion of the search | Examiner |
|---|---|---|
| The Hague | 28 February 2019 | Gabriels, Jan |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO.

EP 18 38 2671

This annex lists the patent family members relating to the patent documents cited in the above-mentioned European search report. The members are as contained in the European Patent Office EDP file on The European Patent Office is in no way liable for these particulars which are merely given for the purpose of information.

28-02-2019

| Patent document cited in search report | Publication date | Patent family member(s) | Publication date |
|---|---|---|---|
| EP 2735617 A1 | 28-05-2014 | AU 2012285223 A1 | 30-01-2014 |
| | | AU 2017261606 A1 | 15-02-2018 |
| | | BR 112014001258 A2 | 21-02-2017 |
| | | CA 2841060 A1 | 24-01-2013 |
| | | CN 103890190 A | 25-06-2014 |
| | | CN 106434865 A | 22-02-2017 |
| | | EP 2735617 A1 | 28-05-2014 |
| | | EP 3354749 A1 | 01-08-2018 |
| | | HK 1199069 A1 | 19-06-2015 |
| | | JP 2017158570 A | 14-09-2017 |
| | | JP WO2013011734 A1 | 23-02-2015 |
| | | US 2014206005 A1 | 24-07-2014 |
| | | US 2016208326 A1 | 21-07-2016 |
| | | WO 2013011734 A1 | 24-01-2013 |
| WO 2018147438 A1 | 16-08-2018 | JP 2018130036 A | 23-08-2018 |
| | | WO 2018147438 A1 | 16-08-2018 |
| WO 2018058114 A1 | 29-03-2018 | NONE | |
| US 2008172209 A1 | 17-07-2008 | US 2008172209 A1 | 17-07-2008 |
| | | US 2008172351 A1 | 17-07-2008 |
| WO 2010019550 A2 | 18-02-2010 | AU 2009282114 A1 | 18-02-2010 |
| | | AU 2016204678 A1 | 21-07-2016 |
| | | AU 2018202373 A1 | 26-04-2018 |
| | | BR PI0917948 A2 | 20-06-2017 |
| | | CA 2735578 A1 | 18-02-2010 |
| | | CN 102177436 A | 07-09-2011 |
| | | CN 104611421 A | 13-05-2015 |
| | | CN 104805198 A | 29-07-2015 |
| | | CO 6351823 A2 | 20-12-2011 |
| | | CY 1115412 T1 | 04-01-2017 |
| | | DK 2324126 T3 | 16-06-2014 |
| | | DO P2011000052 A | 15-04-2011 |
| | | EA 201170332 A1 | 31-10-2011 |
| | | EP 2324126 A2 | 25-05-2011 |
| | | EP 2789695 A1 | 15-10-2014 |
| | | ES 2463766 T3 | 29-05-2014 |
| | | HK 1153511 A1 | 10-10-2014 |
| | | HR P20140709 T1 | 26-09-2014 |
| | | IL 211140 A | 30-06-2015 |
| | | JP 5833922 B2 | 16-12-2015 |
| | | JP 2012500004 A | 05-01-2012 |
| | | JP 2016047058 A | 07-04-2016 |
| | | JP 2018075005 A | 17-05-2018 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 18 38 2671

This annex lists the patent family members relating to the patent documents cited in the above-mentioned European search report. The members are as contained in the European Patent Office EDP file on
The European Patent Office is in no way liable for these particulars which are merely given for the purpose of information.

28-02-2019

| Patent document cited in search report | Publication date | Patent family member(s) | Publication date |
| --- | --- | --- | --- |
| | | KR 20110063453 A | 10-06-2011 |
| | | KR 20160127181 A | 02-11-2016 |
| | | KR 20180053432 A | 21-05-2018 |
| | | MA 32619 B1 | 01-09-2011 |
| | | ME 01898 B | 20-12-2014 |
| | | NZ 591107 A | 31-08-2012 |
| | | PE 03622015 A1 | 20-03-2015 |
| | | PE 05652011 A1 | 02-09-2011 |
| | | PE 19242015 A1 | 13-01-2016 |
| | | PT 2324126 E | 02-06-2014 |
| | | RS 53383 B | 31-10-2014 |
| | | SG 193793 A1 | 30-10-2013 |
| | | SI 2324126 T1 | 31-07-2014 |
| | | SM T201400088 B | 08-09-2014 |
| | | US 2011189165 A1 | 04-08-2011 |
| | | US 2015073025 A1 | 12-03-2015 |
| | | US 2018363060 A1 | 20-12-2018 |
| | | WO 2010019550 A2 | 18-02-2010 |
| | | ZA 201101095 B | 25-07-2012 |

EPO FORM P0459

For more details about this annex : see Official Journal of the European Patent Office, No. 12/82

page 2 of 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 3006571 A1
- EP 2735617 A1
- WO 2015200701 A2
- WO 2014116729 A2
- WO 2014065410 A1
- EP 2599877 A1
- WO 2016054135 A1
- WO 2014150924 A2
- WO 2014066217 A1
- EP 3075863 A1


### Non-patent literature cited in the description


- **MCKENNA, A. et al.** Genome Analysis Tool Kit. Broad Institute, 2010
- algorithm was used for this phase. **MCKENNA, A. et al.** GATK (Genome Analysis Tool_Kit). The Broad Institute, 2010
- **PATTERSON, M. et al.** *WhatsHap software,* 2015, https://whatshap.readthedocs.io/en/latest/
- **BAUER, D. F.** Constructing confidence sets using rank statistics. *Journal of the American Statistical Association,* 1972, vol. 67, 687-690
- **FELSENSTEIN, J.** Evolutionary trees from DNA sequences: a maximum likelihood approach. *Journal of Molecular Evolution,* 1981, vol. 17 (6), 368-376
- **GASCUEL, O. ; BIRKIN.** Concerning the NJ algorithm and its unweighted version, UNJ. *Mathematical Hierarchies and Biology,* 1997, 149-170
- **GU, X. et al.** Locus Specificity of Polymorphic Alleles and Evolution by a Birth-and-Death Process in Mammalian MHC Genes. *Mol Biol Evol,* 1999, vol. 16 (2), 147-56
- **HOSOMICHI, K. et al.** Phase-defined complete sequencing of the HLA genes by next-generation sequencing. *BMC Genomics,* 28 May 2013, vol. 14, 355
- **KATOH, K. et al.** MAFFT Multiple Sequence Alignment Software Version 7: Improvements in Performance and Usability. *Mol Biol Evol,* 2013, vol. 30 (4), 772-80
- **LI, H. et al.** Fast and accurate short read alignment with Burrows-Wheeler transform. *Bioinformatics,* 2009, vol. 25 (14), 1754-1760
- **MCKENNA, A. et al.** The Genome Analysis Toolkit: a MapReduce framework for analyzing next-generation DNA sequencing data. *Genome Res.,* 2010, vol. 20 (9), 1297-303
- **MCKENZIE, L. M. et al.** Taxonomic hierarchy of HLA class I allele sequences. *Genes Immun,* 1999, vol. 1 (2), 120-9
- **PATTERSON, M. et al.** WhatsHap: Weighted Haplotype Assembly for Future-Generation Sequencing Reads. *J. Comput. Biol.,* 2015, vol. 22 (6), 498-509
- **PEARSON, W. R.** Finding Protein and Nucleotide Similarities with FASTA. *Curr Protoc Bioinformatics,* 2016, vol. 53, 3.9.1-25
- **ROUSSEEUW, P. J.** Silhouettes: a Graphical Aid to the Interpretation and Validation of Cluster Analysis. *Journal of Computational and Applied Mathematics,* 1987, vol. 20, 53-65
- **SAITOU, N. et al.** The neighbor-joining method: a new method for reconstructing phylogenetic trees. *Molecular Biology and Evolution,* 1987, vol. 4, 406-425
- **SCHLIEP K. P.** phangorn: phylogenetic analysis. *R. Bioinformatics,* 2011, vol. 27 (4), 592-593
- **SCHLIEP, K. P. et al.** Intertwining phylogenetic trees and networks. *Methods in Ecology and Evolution,* 2017, vol. 8, 1212-1220
- **SHIINA, T. et al.** Super high resolution for single molecule-sequence-based typing of classical HLA loci at the 8-digit level using next generation sequencers. *Tissue Antigens,* 2012, vol. 80 (4), 305-16
- **SNEATH, P.H.A. et al.** *Numerical Taxonomy: The Principles and Practice of Numerical Classification,* 1973, 278 ff
- **STUDIER, J. A. et al.** A Note on the Neighbor-Joining Algorithm of Saitou and Nei. *Molecular Biology and Evolution,* 1988, vol. 6, 729-731